(19) Europäisches Patentamt / European Patent Office / Office européen des brevets

(11) **EP 3 285 072 B1**

(12) **EUROPEAN PATENT SPECIFICATION**

(45) Date of publication and mention of the grant of the patent:
**15.04.2020  Bulletin 2020/16**

(51) Int Cl.:
***G01N 33/92*** (2006.01)

(21) Application number: **17194611.4**

(22) Date of filing: **08.08.2007**

(54) **MARKERS OF NON-ALCOHOLIC FATTY LIVER DISEASE (NAFLD) AND NON-ALCOHOLIC STEATOHEPATITIS (NASH) AND METHODS OF USE THEREOF**

MARKER VON NICHTALKOHOLISCHER FETTLEBERERKRANKUNG (NAFLD) UND NICHTALKOHOLISCHER STEATOHEPATITIS (NASH) SOWIE VERFAHREN ZUR VERWENDUNG DAVON

MARQUEURS DE MALADIE DU FOIE GRAS NON ALCOOLIQUE (NAFLD) ET DE LA STÉATOHÉPATITE NON ALCOOLIQUE (NASH) ET LEURS PROCÉDÉS D'UTILISATION

(84) Designated Contracting States:
**AT BE BG CH CY CZ DE DK EE ES FI FR GB GR HU IE IS IT LI LT LU LV MC MT NL PL PT RO SE SI SK TR**

(30) Priority:  **08.08.2006  US 836555 P**

(43) Date of publication of application:
**21.02.2018  Bulletin 2018/08**

(62) Document number(s) of the earlier application(s) in accordance with Art. 76 EPC:
**12196378.9 / 2 607 902**
**07836680.4 / 2 057 473**

(73) Proprietor: **Metabolon, Inc.**
**Morrisville, NC 27560 (US)**

(72) Inventors:
• **WATKINS, Steven, M.**
**Sacramento, CA 95835 (US)**
• **WIEST, Michelle, M.**
**Knights Landing, CA 95645 (US)**
• **BAILLIE, Rebecca, A.**
**Woodland, CA 95695 (US)**

(74) Representative: **Carpmaels & Ransford LLP**
**One Southampton Row**
**London WC1B 5HA (GB)**

(56) References cited:

WO-A-2006/031963

• PAWLOSKY R J ET AL: "Perspectives on alcohol consumption: liver polyunsaturated fatty acids and essential fatty acid metabolism", ALCOHOL, PERGAMON PRESS, LONDON, GB, vol. 34, no. 1, 1 August 2004 (2004-08-01) , pages 27-33, XP004721101, ISSN: 0741-8329, DOI: 10.1016/J.ALCOHOL.2004.07.009

• ARAYA J ET AL: "Increase in Long-Chain Polyunsaturated Fatty Acid n-6/n-3 Ratio in Relation to Hepatic Steatosis in Patients With Non-Alcoholic Fatty Liver Disease", CLINICAL SCIENCE, BIOCHEMICAL SOCIETY AND THE MEDICAL RESEARCH SOCIETY, LONDON, GB, vol. 106, no. 6, 1 June 2004 (2004-06-01), pages 635-643, XP008104250, ISSN: 0143-5221, DOI: 10.1042/CS20030326 [retrieved on 2004-01-13]

• DE ALMEIDA I T ETA L: "Plasma total and free fatty acids composition in human non-alcoholic steatohepatitis", CLINICAL NUTRITION, CHURCHILL LIVINGSTONE, LONDON, GB, vol. 21, no. 3, 1 January 2002 (2002-01-01), pages 219-223, XP002990383, ISSN: 0261-5614, DOI: 10.1054/CLNU.2001.0529

• PURI P ET AL: "7088 A lipidomic analysis of non-alcoholic fatty liver disease (NAFLD)", JOURNAL OF HEPATOLOGY, MUNKSGAARD INTERNATIONAL PUBLISHERS, COPENHAGEN, DK, vol. 44, 1 April 2006 (2006-04-01), pages S260-S261, XP025054055, ISSN: 0168-8278 [retrieved on 2006-04-01]

EP 3 285 072 B1

**Description**

BACKGROUND OF THE INVENTION

Non-alcoholic steatohepatitis (NASH) is the most common chronic liver disease in the United States. NASH is a fatty inflammation of the liver and a major cause of cirrhosis, fibrosis and liver failure. The disease is progressive, starting as steatosis or nonalcoholic fatty liver disease (NAFLD), progressing to an inflamed fatty liver (NASH), and eventually leading to cirrhosis and fibrosis. The disease is generally asymptomatic until severe liver impairment occurs. The diagnosis of NAFLD or NASH requires liver biopsy as there are no laboratory tests for either of these diseases. The diagnosis of NASH requires the presence of fat, inflammation, and centrolobular (zone 3) ballooning degeneration with either pericellular fibrosis or Mallory bodies. This distinction is important because NASH is believed to be a progressive liver disease which can lead to cirrhosis and even hepatocellular carcinoma.

The prevalence of NAFLD in the U.S. population is -20-23%, and may be as high as 33%, and the prevalence of NASH in the U.S. population is 2-3%. Some NASH patients will progress to late stage disease: approximately 15-50% of NASH patients progress to severe fibrosis, and approximately 7-16% progress to cirrhosis. The rate of liver-specific mortality in NASH cirrhotics is approximately 10% per decade.

Serum aminotransferase elevations and hepatic imaging studies showing changes suggestive of fatty liver are not adequate alone or in combination to distinguish NAFLD from NASH. It is difficult to evaluate the natural history and course of NAFLD or better define its need for therapy or intervention. The causes of NAFLD and NASH are not well defined, but they typically occur in association with obesity, insulin resistance or type II diabetes, and hyperlipidemia, suggesting that fatty liver and NASH are hepatic manifestations of the dysmetabolic syndrome, and might better be referred to as metabolic steatohepatitis (MESH).

The liver is the principal metabolic organ for all lipid metabolic pathways. Under normal conditions, the liver regulates blood lipid levels and manages complex lipid biosynthesis and transport consistent with the energy balance in the body. Thus, liver damage and dysfunction can lead to severe consequences at the organism level. NAFLD has been traditionally viewed to be a benign disease, but a subset of patients will progress to NASH and end-stage liver disease requiring a liver transplant. Because NAFLD is a silent disease, diagnosis at present can be made only through needle biopsy. If recognized, treatment methods for NAFLD and NASH can slow or reverse the disease in some individuals, particularly in early stage disease.

What is needed are better testing methods for diagnosing NAFLD and NASH, monitoring disease progression, and determining efficacy of treatment. Additionally, what is needed are better testing methods that can be used to classify and differentiate between patients with NAFLD and NASH, and to identify patients at risk of transitioning from NAFLD to NASH.

Pawlosky et al. (Alcohol, 2004, 34(1):27-33) reports on the measurement of the concentration of several polyunsaturated fatty acids in total lipid extracts from the livers of subjects diagnosed with alcoholic liver disease.

Araya et al. (Clin. Sci., 2004, 106(6):635-43) provides an assessment of the levels of PUFA (polyunsaturated fatty acids) in liver total lipids, triacylglycerols (triglycerides) and phospholipids of NAFLD patients in relation to those in adipose tissue and hepatic indexes related to oxidative stress as factors contributing to hepatic steatosis.

De Ameida et al. (Clin. Nutr., 2002, 21(3):219-23) evaluated plasma total (esterified plus free) and free fatty acids concentrations in NASH patients.

WO 2006/031963 provides methods of using certain metabolite markers for predicting weight development or its related conditions of a subject.

Puri et al. (Hepatology, 2007, 46(4):1081-90) compares the free fatty acid (FFA), diacylglycerol (DAG), triacylglycerol (TAG), free cholesterol (FC), cholesterol ester, and phospholipid content of normal livers to those of subjects with NAFLD and NASH.

BRIEF SUMMARY OF THE INVENTION

The invention provides a method of diagnosing or monitoring non-alcoholic steatohepatitis (NASH) in a subject, comprising:

(A) determining an amount of one or more lipid metabolites in one or more blood, serum, or plasma samples from the subject, wherein:

the lipid metabolites PC22:6n3, PCLC, PC18:3n3, TG18:3n3, TG20:3n9, TG22:6n3, TG24:0, PE22:6n3, LY22:6n3, PE14:0, PELC, FA16:0, CE22:6n3, LY18:ln9, LY18:2n6, LY18:3n3, and/or 19,20-DiHDPA are negatively associated with NASH; and
the lipid metabolites PC22:5n6, PC18:3n6, PC20:3n6, PC20:4n3, PC22:ln9, PCdm18:1n7, PC18:0, PC22:5n3, CE18:0, CE24:0, CE18:1n7, CE18:3n6, TG18:1n7, LY16:0, FA18:1n7, SM18:0, SM22:1n9, CE20:3n6, TG20:3n6, LY18:0, LY20:3n6, PE18:3n6, PE20:3n6, PE22:5n3, FA18:1n9, FA20:3n6, 15-HETE, PGB2, PGE2, PGF2$\alpha$, 15-keto-PGF2$\alpha$, 5-HETE, 8-HETE, 9-HETE, 11-HETE, 12-HETE, 12-HEPE, 11,12-EpETrE, and/or 8,9-DiHETrE, are positively associated with NASH; and

(B) correlating the amount(s) of the one or more lipid metabolites with the presence, absence or progression of non-alcoholic steatohepatitis (NASH).

In some aspects, disclosed herein are methods of assessing the level of accumulation of triglycerides in the liver of a subject (e.g., a human) and/or monitoring, diagnosing, classifying, assessing the severity, and/or assessing the progression or regression of a liver disorder in the subject. In some embodiments, the liver disorder is hepatic impairment, hepatic steatosis, non-alcoholic fatty liver disease (NAFLD), steatohepatitis, or non-alcoholic steatohepatitis (NASH). In some embodiments, the methods comprise determining the amount of one or more lipid metabolites (e.g., fatty acids and/or eicosanoids) in a body fluid from the subject.

In one aspect, disclosed herein is a method of diagnosing or monitoring a liver disorder in a subject wherein the method comprises determining an amount of one or more lipid metabolites in one or more samples from a body fluid of the subject, and correlating the amount(s) of the one or more lipid metabolites with the presence of the liver disorder. In some embodiments, the lipid metabolites comprise fatty acids and/or eicosanoids. In some embodiments, the liver disorder is hepatic impairment, hepatic steatosis, non-alcoholic fatty liver disease (NAFLD), steatohepatitis, or non-alcoholic steatohepatitis (NASH). In some embodiments, the one or more lipid metabolites are selected from the group consisting of: PC18:3n6; PC20:3n6; CE14:0; CE16:1n7; CE18:1n9; CEMUFA; CEn7; CE18:1n7; CE18:2n6; CE18:3n6; CE22:5n3; CEn6; CEPUFA; PC14:0; PC16:1n7; PC18:1n9; PCI8:3n3; PCI8:4n3; PC20:0; PC20:1n9; PC20:4n3; PC20:5n3; PC22:0; PC22:ln9; PC24:0; PC24:1n9; PCdm; PCdm18:0; PCdm18:1n7; PCSFA; TG14:0; TG14:1n5; TG16:0; TG16:1n7; TG18:1n7; TGMUFA; TGn7; TGSFA; TL14:0; TL16:0; TL18:0; TL16:1n7; TL18:1n7; TL18:1n9; TL18:3n6; TL18:4n3; TG18:3n3; TG20:3n9; TG22:6n3; TG24:0; CE14:1n5; CE18:0; CE20:0; CE20:1n9; CE20:3n9; CE20:4n3; CE20:4n6; CE20:2n6; CE22:0; CE22:2n6; CE24:0; CESFA; PC20:4n6; PC22:5n6; PCn6; PCPUFA; PE20:4n6; TG15:0; TG18:2n6; TG20:0; TG20:2n6; TG20:4n6; TG20:5n3; TG22:0; TG22:2n6; TG22:1n9; TG22:4n6; TG22:5n6; TG24:1n9; TGn3; TGn6; TGPUFA; TL15:0; TL20:0; TL22:0; TL18:2n6; TL20:2n6; TL20:3n9; TL20:4n3; TL20:4n6; TL22:4n6; TL22:5n6; LY16:0; FA18:1n7; SM18:0; SM22:1n9; SMLC; PGB2; PGE2; PGF2$\alpha$; 15-keto-PGF2$\alpha$; 5-HETE; 8-HETE; 9-HETE; 11-HETE; 12-HETE; 12-HEPE; 11,12-EpETrE; 8,9-DiHETrE; PC18:0; PC22:5n3; CE20:3n6; CELC; TGLC; TG18:3n6; TG20:4n3; TG20:3n6; TG22:5n3;LYLC; LY18:0; LY20:3n6; PE18:3n6; PE20:3n6; PE22:5n3; FA18:0; FA20:5n3; FA18:1n9; FA20:3n6; 15-HETE; TL20:3n6; PC18:2n6; PC20:2n6; PE20:2n6; SM16:0; PGA2M; 6-keto-PGF1$\alpha$; 11-DTXB2; 12,13-DiHOME; 9,10-EpOME; 12,13-EpOME; PC22:6n3; PE22:6n3; LY22:6n3; PE14:0; PE18:1n7; PESFA; PELC; FA16:0; CE22:6n3, TL22:6n3; PCLC; PC18:1n7; LY18:1n7; LY18:1n9; LY18:2n6; LY18:3n3; and 19,20-DiHDPA. In some embodiments where the one or more lipid metabolites comprise one or more fatty acids, the amount(s) of the one or more fatty acids are the relative amount(s) of the one or more fatty acids to total fatty acid content in the lipids of one or more lipid classes in one or more samples. The methods can, in some embodiments, further comprise comparing the amount(s) of the one or more lipid metabolites to one or more references (e.g., a normal control). In some embodiments, the amounts of two or more, three or more, four or more, five or more, or six or more lipid metabolites are determined. In some embodiments, the sample(s) are selected from the group consisting of blood, plasma, serum, isolated lipoprotein fraction, saliva, urine, lymph fluid, and cerebrospinal fluid.

Also disclosed herein is a method of diagnosing or monitoring a liver disorder in a subject, which comprises determining a relative amount of one or more fatty acids to total fatty acid content in the lipids of one or more lipid classes in a sample from a body fluid of the subject, and correlating the relative amount(s) with the presence of the liver disorder; wherein the liver disorder is hepatic impairment, hepatic steatosis, non-alcoholic fatty liver disease (NAFLD), steatohepatitis, or non-alcoholic steatohepatitis (NASH). In some embodiments, the one or more fatty acids are selected from the group consisting of: PC 18:3n6; PC20:3n6; CEI 4:0; CE 16: 1 n7; CE 18: 1 n9; CEMUFA; CEn7; CE 18: 1 n7; CE 18:2n6; CE18:3n6; CE22:5n3; CEn6; CEPUFA; PC 14:0; PC 16: 1 n7; PC18:ln9; PC18:3n3; PC18:4n3; PC20:0; PC20:ln9; PC20:4n3; PC20:5n3; PC22:0; PC22:ln9; PC24:0; PC24:ln9; PCdm; PCdm18:0; PCdm18:ln7; PCSFA; TG14:0; TG14:ln5; TG16:0; TG16:ln7; TG18:ln7; TGMUFA; TGn7; TGSFA; TL14:0; TL 16:0; TL18:0; TL16:ln7; TL18:ln7; TL18:ln9; TL18:3n6; TL18:4n3; TG18:3n3; TG20:3n9; TG22:6n3; TG24:0; CE14:ln5; CE18:0; CE20:0; CE20:ln9; CE20:3n9; CE20:4n3; CE20:4n6; CE20:2n6; CE22:0; CE22:2n6; CE24:0; CESFA; PC20:4n6; PC22:5n6; PCn6; PCPUFA; PE20:4n6; TG15:0; TG18:2n6; TG20:0; TG20:2n6; TG20:4n6; TG20:5n3; TG22:0; TG22:2n6; TG22:ln9; TG22:4n6; TG22:5n6; TG24:ln9; TGn3; TGn6; TGPUFA; TL15:0; TL20:0; TL22:0; TL18:2n6; TL20:2n6; TL20:3n9; TL20:4n3; TL20:4n6; TL22:4n6; and TL22:5n6. In some embodiments, the method comprises the step of comparing the relative amount of one or more fatty acids to a reference. In some embodiments, the liver disorder is NASH, and the method further comprises the step of determining the level of an eicosanoid in a body fluid. In some embodiments, the relative amounts of two or more, three or more, four or more, five or more, or six or more fatty acids are determined. In some embodiments, the sample is selected from the group consisting of blood, plasma, serum, isolated lipoprotein fraction, saliva, urine, lymph fluid, and cerebrospinal fluid.

Also disclosed herein is a method of assessing the level of triglycerides in the liver of a subject, comprising determining the amount of a lipid metabolite in a sample from a body fluid of the subject, wherein the lipid metabolite is a fatty acid present in a lipid class, and wherein the lipid class is selected from the group consisting of free fatty acids, total fatty acids, triglycerides, cholesterol esters, phosphatidylcholines, and phosphatidylethanolamines. In some embodiments, the amount of the metabolite is the relative amount of the fatty acid to total fatty acid content in the lipids of one or more lipid classes in the sample. In some embodiments, the fatty acid is selected from the group consisting of: PC18:3n6; PC20:3n6; CE 14:0; CE16:ln7; CE18:ln9; CEMUFA; CEn7; CE18:ln7; CE18:2n6; CE18:3n6; CE22:5n3; CEn6; CEPUFA; PC14:0; PC16:ln7; PC18:ln9; PC 18:3n3; PC18:4n3; PC20:0; PC20:ln9; PC20:4n3; PC20:5n3; PC22:0; PC22:ln9; PC24:0; PC24:ln9; PCdm; PCdm18:0; PCdm18:ln7; PCSFA; TG14:0; TG14:ln5; TG16:0; TG16:ln7; TG18:ln7; TGMUFA; TGn7; TGSFA; TL14:0; TL16:0; TL18:0; TL16:ln7; TL18:ln7; TL18:ln9; TL18:3n6; TL18:4n3; CE14:ln5; CE18:0; CE20:0; CE20:ln9; CE20:3n9; CE20:4n3; CE20:4n6; CE20:2n6; CE22:0; CE22:2n6; CE24:0; CESFA; PC20:4n6; PC22:5n6; PCn6; PCPUFA; PE20:4n6; TG 15:0; TG18:2n6; TG20:0; TG20:2n6; TG20:4n6; TG20:5n3; TG22:0; TG22:2n6; TG22: ln9; TG22:4n6; TG22:5n6; TG24:ln9; TGn3; TGn6; TGPUFA; TL 15:0; TL20:0; TL22:0; TL18:2n6; TL20:2n6; TL20:3n9; TL20:4n3; TL20:4n6; TL22:4n6; and TL22:5n6. In some embodiments, the method further comprises the step of comparing the relative amount of one or more fatty acid to a reference. In some embodiments, the sample is selected from the group consisting of blood, plasma, serum, isolated lipoprotein fraction, saliva, urine, lymph fluid, and cerebrospinal fluid.

Also disclosed herein are methods of assessing the level of triglycerides in the liver of a subject, comprising determining the amount of a lipid metabolite in a sample from a body fluid of the subject. In some embodiments, the method comprises determining the amount of at least 2, at least 3, at least 4, at least 5, at least 10, at least 15, or at least 20 lipid metabolites. In some embodiments, the lipid metabolite is a fatty acid present in a lipid class. In some embodiments, the lipid class is selected from the group consisting of: free fatty acids, total fatty acids, triglycerides, cholesterol esters, phosphatidylcholines, and phosphatidylethanolamines. In some embodiments, the lipid class is selected from the group consisting of: neutral lipids, free fatty acids, total fatty acids, triglycerides, cholesterol esters, phospholipids, phosphatidylcholines, and phosphatidylethanolamines. In some embodiments, the lipid class is selected from the group consisting of: neutral lipids, total fatty acids, cholesterol esters, and phospholipids. In some embodiments, the amount of the metabolite is the relative amount of a fatty acid to total fatty acid content in the lipids of one or more lipid classes in the sample. In some embodiments, the relative amount is selected from the group consisting of: (a) the relative amount of a fatty acid to total fatty acid content in triglycerides in the sample; (b) the relative amount of a fatty acid to total fatty acid content in free fatty acids in the sample; (c) the relative amount of a fatty acid to total fatty acid content in phosphatidylcholines in the sample; (d) the relative amount of a fatty acid to total fatty acid content in phosphatidylethanolamines in the sample; (e) the relative amount of a fatty acid to total fatty acid content in cholesterol esters in the sample; and (f) the relative amount of a fatty acid to total fatty acid content in all lipids in the sample. In some embodiments, the fatty acid is selected from the group consisting of: TG14:0, TG14:1n5, TG16:0, TG18:1n7, TGMUFA, TGn7, TGSFA, TG16:1n7, PC14:0, PC16:1n7, PC18:1n7, PC18:1n9, PC18:3n3, PC18:3n6, PC18:4n3, PC20:0, PC20:1n9, PC20:2n6, PC20:3n6, PC20:4n3, PC20:5n3, PC22:0, PC22:1n9, PC24:0, PC24:1n9, PCdm, PCdm 18:0, PCdm18:1n7, PCSFA, CE16:1n7, CE18:1n7, CE18:1n9, CE18:2n6, CE18:3n6, CE22:5n3, CE22:6n3, CEMUFA, CEn6, CEn7, CEPUFA, CE14:0, 14:0, 16:0, 18:0, 16:1n7, 18:1n7, 18:1n9, 18:3n6, 18:4n3, TG15:0, TG18:2n6, TG18:3n3, TG20:0, TG20:2n6, TG20:3n6, TG20:3n9, TG20:4n6, TG20:5n3, TG22:0, TG22:1n9, TG22:2n6, TG22:4n6, TG22:5n3, TG22:5n6, TG22:6n3, TG24:0, TG24:1n9, TGn3, TGn6, TGPUFA, FA16:1n7, PC18:1n7, PC20:4n6, PC22:5n6, PCn6, PCPUFA, PC22:5n3, PE20:4n6, CE14:1n5, CE18:0, CE20:0, CE20:1n9, CE20:2n6, CE20:3n9, CE20:4n3, CE20:4n6, CE22:0, CE22:2n6, CE24:0, CESFA, 15:0, 20:0, 22:0, 18:2n6, 20:2n6, 20:3n9, 20:4n3, 20:4n6, 22:4n6, and 22:5n6. In some embodiments, the fatty acid is TG20:4n6. In some embodiments, the sample is selected from the group consisting of blood, plasma, serum, isolated lipoprotein fraction, saliva, urine, lymph fluid, and cerebrospinal fluid. In some embodiments, the sample is selected from the group consisting of blood, plasma, serum, or isolated lipoprotein fraction. In some embodiments, the sample is lymph or cerebrospinal fluid.

In some embodiments, the level of accumulation of triglycerides in the liver of a subject is assessed, comprising determining a relative amount of one or more fatty acids to total fatty acid content in triglycerides in a sample from a body fluid of the subject. In some embodiments, the one or more fatty acids are selected from the group consisting of TG14:0, TG14:1n5, TG16:0, TG18:1n7, TGMUFA, TGn7, TGSFA, and TG16:1n7. The method may further comprise the step of comparing the relative amount to a reference, wherein if the relative amount is greater than the reference, accumulation of triglycerides in the liver is indicated. In some embodiments, the one or more fatty acids are selected from the group consisting of TG15:0, TG18:2n6, TG18:3n3, TG20:0, TG20:2n6, TG20:3n6, TG20:3n9, TG20:4n6, TG20:5n3, TG22:0, TG22:1n9, TG22:2n6, TG22:4n6, TG22:5n3, TG22:5n6, TG22:6n3, TG24:0, TG24:1n9, TGn3, TGn6, and TGPUFA. The method may further comprise the step of comparing the relative amount to a reference, wherein if the relative amount is lower than the reference, accumulation of triglycerides in the liver is indicated. In some embodiments, the reference is a relative amount of the one or more fatty acids to total fatty acid content in the triglycerides in a sample from a body fluid previously obtained from the subject. In some embodiments, the reference represents the relative amount of the one or more fatty acids to total fatty acid content in the triglycerides found in one or more samples from a body fluid of one or more subjects having normal livers.

In some embodiments, the level of accumulation of triglycerides in the liver of a subject is assessed, comprising determining a relative amount of a fatty acid to total fatty acid content in free fatty acids in a sample from a body fluid of the subject. In some embodiments, the fatty acid is FA16:1n7. The method may further comprise the step of comparing the relative amount to a reference, wherein if the relative amount is lower than the reference, accumulation of triglycerides in the liver is indicated. In some embodiments, the reference is a relative amount of the one or more fatty acids to total fatty acid content in the free fatty acids in a sample from a body fluid previously obtained from the subject. In some embodiments, the reference represents the relative amount of the one or more fatty acids to total fatty acid content in the free fatty acids found in one or more samples from a body fluid of one or more subjects having normal livers.

In some embodiments, the level of accumulation of triglycerides in the liver of a subject is assessed, comprising determining a relative amount of one or more fatty acids to total fatty acid content in phosphatidylcholines in a sample from a body fluid of the subject. In some embodiments, the one or more fatty acids are selected from the group consisting of PC14:0, PC16:1n7, PC18:1n7, PC18:1n9, PC18:3n3, PC18:3n6, PC18:4n3, PC20:0, PC20:1n9, PC20:2n6, PC20:3n6, PC20:4n3, PC20:5n3, PC22:0, PC22:1n9, PC24:0, PC24:1n9, PCdm, PCdm18:0, PCdm18:1n7, and PCSFA. The method may further comprise the step of comparing the relative amount to a reference, wherein if the relative amount is greater than the reference, accumulation of triglycerides in the liver is indicated. In some embodiments, the one or more fatty acids are selected from the group consisting of PC18:1n7, PC20:4n6, PC22:5n6, PCn6, PCPUFA, and PC22:5n3. The method may further comprise the step of comparing the relative amount to a reference, wherein if the relative amount is lower than the reference, accumulation of triglycerides in the liver is indicated. In some embodiments, the reference is a relative amount of the one or more fatty acids to total fatty acid content in the phosphatidylcholines in a sample from a body fluid previously obtained from the subject. In some embodiments, the reference represents the relative amount of the one or more fatty acids to total fatty acid content in the phosphatidylcholines found in one or more samples from a body fluid of one or more subjects having normal livers.

In some embodiments, the level of accumulation of triglycerides in the liver of a subject is assessed, comprising determining a relative amount of a fatty acid to total fatty acid content in phosphatidylethanolamines in a sample from a body fluid of the subject. In some embodiments, the fatty acid is PE20:4n6. The method may further comprise the step of comparing the relative amount to a reference, wherein if the relative amount is lower than the reference, accumulation of triglycerides in the liver is indicated. In some embodiments, the reference is a relative amount of the one or more fatty acids to total fatty acid content in the phosphatidylethanolamines in a sample from a body fluid previously obtained from the subject. In some embodiments, the reference represents the relative amount of the one or more fatty acids to total fatty acid content in the phosphatidylethanolamines found in one or more samples from a body fluid of one or more subjects having normal livers.

In some embodiments, the level of accumulation of triglycerides in the liver of a subject is assessed, comprising determining a relative amount of one or more fatty acids to total fatty acid content in a sample from a body fluid of the subject. In some embodiments, the one or more fatty acids are selected from the group consisting of 14:0, 16:0, 18:0, 16:1n7, 18:1n7, 18:1n9, 18:3n6, and 18:4n3. The method may further comprise the step of comparing the relative amount to a reference, wherein if the relative amount is greater than the reference, accumulation of triglycerides in the liver is indicated. In some embodiments, the one or more fatty acids are selected from the group consisting of 15:0, 20:0, 22:0, 18:2n6, 20:2n6, 20:3n9, 20:4n3, 20:4n6, 22:4n6, and 22:5n6. The method may further comprise the step of comparing the relative amount to a reference, wherein if the relative amount is lower than the reference, accumulation of triglycerides in the liver is indicated. In some embodiments, the reference is a relative amount of the one or more fatty acids to total fatty acid content in a sample from a body fluid previously obtained from the subject. In some embodiments, the reference represents the relative amount of the one or more fatty acids to total fatty acid content found in one or more samples from a body fluid of one or more subjects having normal livers.

In some embodiments, the level of accumulation of triglycerides in the liver of a subject is assessed, comprising determining a relative amount of one or more fatty acids to total fatty acid content in cholesterol esters in a sample from a body fluid of the subject. In some embodiments, the one or more fatty acids are selected from the group consisting of CE16:1n7, CE18:1n7, CE18:1n9, CE18:2n6, CE18:3n6, CE22:5n3, CE22:6n3, CEMUFA, CEn6, CEn7, CEPUFA, CE14:0. The method may further comprise the step of comparing the relative amount to a reference, wherein if the relative amount is greater than the reference, accumulation of triglycerides in the liver is indicated. In some embodiments, the one or more fatty acids are selected from the group consisting of CE14:1n5, CE18:0, CE20:0, CE20:1n9, CE20:2n6, CE20:3n9, CE20:4n3, CE20:4n6, CE22:0, CE22:2n6, CE24:0, and CESFA. The method may further comprise the step of comparing the relative amount to a reference, wherein if the relative amount is lower than the reference, accumulation of triglycerides in the liver is indicated. In some embodiments, the reference is a relative amount of the one or more fatty acids to total fatty acid content in the cholesterol esters in a sample from a body fluid previously obtained from the subject. In some embodiments, the reference represents the relative amount of the one or more fatty acids to total fatty acid content in the cholesterol esters found in one or more samples from a body fluid of one or more subjects having normal livers.

In some embodiments, the level of accumulation of triglycerides in the liver of a subject is assessed, comprising determining a relative amount of one or more fatty acids to total fatty acid content in neutral lipids in a sample from a body fluid of the subject. In some embodiments, the one or more fatty acids are selected from the group consisting of TG14:0, TG14:1n5, TG16:0, TG18:1n7, TGMUFA, TGn7, TGSFA, and TG16:1n7. The method may further comprise the step of comparing the relative amount to a reference, wherein if the relative amount is greater than the reference, accumulation of triglycerides in the liver is indicated. In some embodiments, the one or more fatty acids are selected from the group consisting of TG15:0, TG18:2n6, TG18:3n3, TG20:0, TG20:2n6, TG20:3n6, TG20:3n9, TG20:4n6, TG20:5n3, TG22:0, TG22:1n9, TG22:2n6, TG22:4n6, TG22:5n3, TG22:5n6, TG22:6n3, TG24:0, TG24:1n9, TGn3, TGn6, TGPUFA, and FA16:1n7. The method may further comprise the step of comparing the relative amount to a reference, wherein if the relative amount is lower than the reference, accumulation of triglycerides in the liver is indicated. In some embodiments, the reference is a relative amount of the one or more fatty acids to total fatty acid content in the neutral lipids in a sample from a body fluid previously obtained from the subject. In some embodiments, the reference represents the relative amount of the one or more fatty acids to total fatty acid content in the neutral lipids found in one or more samples from a body fluid of one or more subjects having normal livers.

In some embodiments, the level of accumulation of triglycerides in the liver of a subject is assessed, comprising determining a relative amount of one or more fatty acids to total fatty acid content in phospholipids in a sample from a body fluid of the subject. In some embodiments, the one or more fatty acids are selected from the group consisting of PC14:0, PC16:1n7, PC18:1n7, PC18:1n9, PC18:3n3, PC18:3n6, PC18:4n3, PC20:0, PC20:1n9, PC20:2n6, PC20:3n6, PC20:4n3, PC20:5n3, PC22:0, PC22:1n9, PC24:0, PC24:1n9, PCdm, PCdm 18:0, PCdm18:1n7, and PCSFA. The method may further comprise the step of comparing the relative amount to a reference, wherein if the relative amount is greater than the reference, accumulation of triglycerides in the liver is indicated. In some embodiments, the one or more fatty acids are selected from the group consisting of PC18:1n7, PC20:4n6, PC22:5n6, PCn6, PCPUFA, PC22:5n3, and PE20:4n6. The method may further comprise the step of comparing the relative amount to a reference, wherein if the relative amount is lower than the reference, accumulation of triglycerides in the liver is indicated. In some embodiments, the reference is a relative amount of the one or more fatty acids to total fatty acid content in the phospholipids in a sample from a body fluid previously obtained from the subject. In some embodiments, the reference represents the relative amount of the one or more fatty acids to total fatty acid content in the phospholipids found in one or more samples from a body fluid of one or more subjects having normal livers.

In some embodiments, the method further comprises determining at least 1, at least 2, at least 3, at least 4, at least 5, at least 10, or at least 20 additional relative amounts, wherein the relative amount(s) is the relative amount of a fatty acid to total fatty acid content in the lipids of one or more lipid classes in the sample. In some embodiments, the method further comprises determining an additional relative amount, wherein the additional relative amount is selected from the group consisting of: (a) the relative amount of a fatty acid to total fatty acid content in triglycerides in the sample; (b) the relative amount of a fatty acid to total fatty acid content in free fatty acids in the sample; (c) the relative amount of a fatty acid to total fatty acid content in phosphatidylcholines in the sample; (d) the relative amount of a fatty acid to total fatty acid content in phosphatidylethanolamines in the sample; (e) the relative amount of a fatty acid to total fatty acid content in cholesterol esters in the sample; and (f) the relative amount of a fatty acid to total fatty acid content in all lipids in the sample. In some embodiments, the additional relative amount is selected from the group consisting of: (a) a relative amount of one or more fatty acids to total fatty acid content in triglycerides in a sample from a body fluid of the subject, wherein the one or more fatty acids are selected from the group consisting of TG14:0, TG14:1n5, TG16:0, TG18:1n7, TGMUFA, TGn7, TGSFA, and TG16:1n7; (b) a relative amount of one or more fatty acids to total fatty acid content in phosphatidylcholines in a sample from a body fluid of the subject, wherein the one or more fatty acids are selected from the group consisting of PC14:0, PC16:1n7, PC18:1n7, PC18:1n9, PC18:3n3, PC18:3n6, PC 18:4n3, PC20:0, PC20:1n9, PC20:2n6, PC20:3n6, PC20:4n3, PC20:5n3, PC22:0, PC22:1n9, PC24:0, PC24:1n9, PCdm, PCdm18:0, PCdm18:1n7, and PCSFA; (c) a relative amount of one or more fatty acids to total fatty acid content in cholesterol esters in a sample from a body fluid of the subject, wherein the one or more fatty acids are selected from the group consisting of CE16:1n7, CE18:1n7, CE18:1n9, CE18:2n6, CE18:3n6, CE22:5n3, CE22:6n3, CEMUFA, CEn6, CEn7, CEPUFA, CE14:0; and (d) a relative amount of one or more fatty acids to total fatty acid content in a sample from a body fluid of the subject, wherein the one or more fatty acids are selected from the group consisting of 14:0, 16:0, 18:0, 16:1n7, 18:1n7, 18:1n9, 18:3n6, and 18:4n3. The method may further comprise the step of comparing the additional relative amount to an additional reference, wherein if the additional relative amount is greater than the additional reference, accumulation of triglycerides in the liver is indicated. In some embodiments, the additional relative amount is selected from the group consisting of: (a) a relative amount of one or more fatty acids to total fatty acid content in triglycerides in a sample from a body fluid of the subject, wherein the one or more fatty acids are selected from the group consisting of TG15:0, TG18:2n6, TG18:3n3, TG20:0, TG20:2n6, TG20:3n6, TG20:3n9, TG20:4n6, TG20:5n3, TG22:0, TG22:1n9,

TG22:2n6, TG22:4n6, TG22:5n3, TG22:5n6, TG22:6n3, TG24:0, TG24:1n9, TGn3, TGn6, and TGPUFA; (b) a relative amount of a fatty acid to total fatty acid content in free fatty acids in a sample from a body fluid of the subject, wherein the fatty acid is FA16:1n7; (c) a relative amount of one or more fatty acids to total fatty acid content in phosphatidylcholines in a sample from a body fluid of the subject, wherein the one or more fatty acids are selected from the group consisting of PC18:1n7, PC20:4n6, PC22:5n6, PCn6, PCPUFA, and PC22:5n3; (d) a relative amount of a fatty acid to total fatty acid content in phosphatidylethanolamines in a sample from a body fluid of the subject, wherein the fatty acid is PE20:4n6; and (e) a relative amount of a fatty acid to total fatty acid content in cholesterol esters in a sample from a body fluid of the subject, wherein the one or more fatty acids are selected from the group consisting of CE14:1n5, CE18:0, CE20:0, CE20:1n9, CE20:2n6, CE20:3n9, CE20:4n3, CE20:4n6, CE22:0, CE22:2n6, CE24:0, CESFA; and (f) a relative amount of one or more fatty acids to total fatty acid content in a sample from a body fluid of the subject, wherein the one or more fatty acids are selected from the group consisting of 15:0, 20:0, 22:0, 18:2n6, 20:2n6, 20:3n9, 20:4n3, 20:4n6, 22:4n6, and 22:5n6. The method may further comprise the step of comparing the additional relative amount to a reference, wherein if the relative amount is lower than the reference, accumulation of triglycerides in the liver is indicated.

Methods of assessing the level of triglycerides in the liver of a subject may be used in diagnosing, monitoring, assessing the severity, and/or assessing the progression or regression of a liver disorder, wherein the liver disorder is selected from the group consisting of: hepatic impairment, hepatic steatosis, NAFLD, steatohepatitis, and NASH. In some embodiments, the method of diagnosing a liver disorder in a subject comprises (a) determining a relative amount of one or more fatty acids to total fatty acid content in the lipids of one or more lipid classes in a sample from a body fluid of the subject; (b) correlating the relative amount with the presence of the liver disorder; and wherein the liver disorder is hepatic impairment, hepatic steatosis, NAFLD, steatohepatitis, or NASH. In some embodiments, the method of assessing the severity of a liver disorder in a subject comprises (a) determining a relative amount of one or more fatty acids to total fatty acid content in the lipids of one or more lipid classes in a sample from a body fluid of the subject; (b) correlating the relative amount with severity of the liver disorder; and wherein the liver disorder is hepatic impairment, hepatic steatosis, NAFLD, steatohepatitis, or NASH. In some embodiments, the method of monitoring a liver disorder in a subject comprises (a) determining a relative amount of one or more fatty acids to total fatty acid content in the lipids of one or more lipid classes in a sample from a body fluid of the subject; (b) correlating the relative amount with the state of the liver disorder; and wherein the liver disorder is hepatic impairment, hepatic steatosis, NAFLD, steatohepatitis, or NASH. In some embodiments, the method of assessing the progression or regression of a liver disorder in a subject comprises (a) determining a relative amount of one or more fatty acids to total fatty acid content in the lipids of one or more lipid classes in a sample from a body fluid of the subject; (b) correlating the relative amount with the state of the liver disorder; and wherein the liver disorder is hepatic impairment, hepatic steatosis, NAFLD, steatohepatitis, or NASH. In some embodiments, the relative amount is measured at two or more time points. In some embodiments, the method of monitoring, assessing the severity, or assessing the progression or regression of the liver disorder is used to determine the subject's response to treatment. In some embodiments, the method may further comprise the step of comparing the relative amount to a reference, wherein if the relative amount is greater than the reference, hepatic impairment, hepatic steatosis, NAFLD, steatohepatitis, or NASH is indicated. In some embodiments, the method may comprise the step of comparing the relative amount to a reference, wherein if the relative amount is lower than the reference, hepatic impairment, hepatic steatosis, NAFLD, steatohepatitis, or NASH is indicated. In some embodiments, the method may further comprise the step of determining an additional relative amount of one or more fatty acids to total fatty acid content in the lipids of one or more lipid classes in a sample from a body fluid of the subject. In some embodiments, the liver disorder is associated with one or more conditions selected from the group consisting of: hepatitis, HIV infection, HBV infection, HCV infection, viral-induced steatosis, and steatosis induced by a non-viral infectious agent. In some embodiments, the liver disorder is associated with drug-induced steatosis. In some embodiments, the drug-induced steatosis is induced by tamoxifen, an uncoupling protein inhibitor, Isoniazid, Rifampicin, a fibrate, or a peroxisome proliferator-activated receptor (PPAR) agonist. In some embodiments, the liver disorder is associated with one or more conditions selected from the group consisting of: obesity, polycystic ovary syndrome (PCOS), diabetes, insulin resistance, and metabolic disorder. In some embodiments, the liver disorder associated with one or more conditions selected from the group consisting of: alcoholic fatty liver disease and alcoholic steatohepatitis. In some embodiments, the liver disorder is associated with an inborn error of metabolism or a genetic alteration. In some embodiments, the inborn error of metabolism or genetic alteration is selected from the group consisting of citrin deficiency, hemochromatosis, and hyperferritinemia. In some embodiments, the liver disorder is associated with toxin-induced steatosis or toxin-induced steatohepatitis. In some embodiments, the toxin-induced steatosis or toxin-induced steatohepatitis is induced by carbon tetrachloride. In some embodiments, the liver disorder is associated with one or more conditions selected from the group consisting of: malnutrition, impaired nutrient absorption, celiac disease, and lipodystrophy. In some embodiments, the liver disorder is associated with bariatric surgery or a liver transplant.

Additional biomarkers and examinations may be used in the methods of diagnosing, monitoring, assessing severity, and for assessing progression or regression of the liver disorder. In some embodiments, the method further comprises: (c) determining the level of malonyl-CoA or malonyl carnitine in a body fluid or cellular sample from the subject, wherein a higher than normal level is indicative of steatosis, NAFLD, or NASH; (d) determining the level of an acylcarnitine, free carnitine, or butyrobetaine in a body fluid or cellular sample from the subject, wherein a lower than normal level is indicative of hepatic impairment, hepatic steatosis, NAFLD, steatohepatitis, or "NASH; and/or (e) determining the level of a sterol or bile acid in a body fluid or cellular sample from the subject, wherein a higher than normal level is indicative of hepatic impairment, hepatic steatosis, NAFLD, steatohepatitis, or NASH. In some embodiments, the acylcarnitine is an acylcarnitine in Table 3. In some embodiments, the sterol or bile acid is a sterol or bile acid in Table 4. In some embodiments, the method further comprises the step of determining the level of an eicosanoid in a body fluid or cellular sample from the subject, wherein a higher than normal level is indicative of NASH. In some embodiments, the eicosanoid is an eicosanoid in Table 2. In some embodiments, the method further comprises the step of determining the level of a cytokine, cytokeratine, chemokine, adipokine, or leptin in a body fluid or cellular sample from the subject. In some embodiments, the cytokine, cytokeratine, chemokine, adipokine, or leptin is TNF, IL-6, CCL2/MCP-1 or CCL19, and a higher than normal level is indicative of NASH. In some embodiments, the cytokine or cytokeratine is IL-8, IL-18, cytokeratine 8 or cytokeratine 18, and a lower than normal level is indicative of NASH. In some embodiments, the method further comprises the step of (a) performing a physical examination of the subject; (b) measuring the level of an aminotransferase in the blood of the subject; or (c) obtaining an image of the liver of the subject.

In some embodiments of each of the aforementioned aspects, as well as other aspects described herein, the subject is a mammal, such as a human. In some embodiments, the mammal is a primate.

In some embodiments of each of the aforementioned aspects, as well as other aspects described herein, the subject is a liver graft donor candidate, is being evaluated for bariatric surgery, has had bariatric surgery, or is being monitored for weight loss.

Also disclosed here in are kits for use in the methods of the invention. In some embodiments, the kit comprises (a) an antibody to the marker (e.g., fatty acid or eicosanoid); and (b) instructions for use. In some embodiments, the kit further comprises: (c) a second antibody to a second marker (e.g., fatty acid or eicosanoid). In some embodiments, the kit further comprises: (d) a third antibody to a third marker (e.g., fatty acid or eicosanoid).

Where aspects or embodiments of the disclosure are described herein in terms of a Markush group or other grouping of alternatives, the present disclosure encompasses not only the entire group listed as a whole, but each member of the group individually and all possible subgroups of the main group, but also the main group absent one or more of the group members. The present disclosure also envisages the explicit exclusion of one or more of any of the group members in the claimed disclosure.

BRIEF DESCRIPTION OF THE DRAWINGS

FIG. 1 shows that a lipid metabolite that is a relative proportion (shown in darker grey) of a triglyceride (or any other lipid class) can be measured in, for example, serum or plasma, as a quantitative measure of the relative proportion of that lipid metabolite in hepatic triglycerides.

FIG. 2 shows the correlation of the fatty acid composition of matched plasma and liver lipid classes from normal subjects.

FIG. 3 shows the relationship between hepatic triglyceride concentrations (nmoles/g) and the relative proportion of lipid 20:4n6 in hepatic triglycerides (expressed as a mole percentage of total triglyceride fatty acids).

FIG. 4 shows the Receiver Operating Characteristic (ROC) curve for Liver TG20:4n6.

DETAILED DESCRIPTION OF THE INVENTION

In some aspects, the invention provides testing methods that can be used to diagnose, classify, and/or monitor patients with NASH associated with increased liver triglyceride levels, and to identify patients at risk of transitioning from steatosis or NAFLD to NASH.

Hepatic triglycerides levels determine the severity of steatosis. Because the accumulation of triglyceride within liver (steatosis) is the result of inadequate export of triglyceride out of liver via very low density lipoprotein (VLDL) secretion, the absolute amount of triglyceride in plasma is not a consistent measure of the magnitude of steatosis. The inventors have discovered that particular amounts of lipid metabolites in body fluids correlate with liver triglyceride levels, independent of the absolute flux of triglycerides from liver into plasma.

With respect to the nomenclature for fatty acid lipid metabolites used herein, fatty acids labeled with a prefix "CE", "DG", "FA", "LY", "PC", "PE", "SM","TG," or "TL" refer to the indicated fatty acids present within cholesterol esters, diglycerides, free fatty acids, lysophosphatidylcholines, phosphatidylchol ines, phosphatidylethanolamines, sphingomyelins, triglycerides, and total lipids, respectively, in a sample. In some embodiments, the indicated fatty acid components are quantified as a proportion of total fatty acids within the lipid class indicated by the prefix. The prefix "SP" is used interchangeably herein with "SM" for fatty acids in sphingomyelins in a sample. References to fatty acids without a prefix or other indication of a particular lipid class generally indicate fatty acids present within total lipids in a sample. The term "LC" following a prefix "CE", "DG", "FA", "LY", "PC", "PE", "SM","TG," or "TL" refers to the amount of the total lipid class indicated by the prefix in the sample (e.g., the concentration of lipids of that class expressed as nMoles per gram of serum or plasma). For example, with respect to a measurement taken from plasma or serum, in some embodiments, the abbreviation "PC18:2n6" indicates the percentage of plasma or serum phosphatidylcholine comprised of linoleic acid (18:2n6), and the term "TGLC" indicates the absolute amount (e.g., in nMoles per gram) of triglyceride present in plasma or serum.

In some embodiments, the liver disorder is steatosis and/or NAFLD and the one or more lipid metabolites are selected from the group consisting of: PC18:3n6; PC20:3n6; CE14:0; CE16:ln7; CE18:1n9; CEMUFA; CEn7; CE18:1n7; CE18:2n6; CE18:3n6; CE22:5n3; CEn6; CEPUFA; PC14:0; PC16:1n7; PC18:1n9; PC18:3n3; PC18:4n3; PC20:0; PC20:1n9; PC20:4n3; PC20:5n3; PC22:0; PC22:1n9; PC24:0; PC24:1n9; PCdm; PCdm18:0; PCdml 8:1n7; PCSFA; TG14:0; TG14:1n5; TG16:0; TG16:1n7; TG18:1n7; TGMUFA; TGn7; TGSFA; TL14:0; TL16:0; TL18:0; TL16:1n7; TL18:1n7; TL18:1n9; TL18:3n6; TL18:4n3; PC18:0; PC22:5n3; CE20:3n6; CELC; TGLC; TG18:3n6; TG20:4n3; TG20:3n6; TG22:5n3;LYLC; LY18:0; LY20:3n6; PE18:3n6; PE20:3n6; PE22:5n3; FA18:0; FA20:5n3; FA18:1n9; FA20:3n6; 15-HETE; TL20:3n6; CE14:1n5; CE18:0; CE20:0; CE20:1n9; CE20:3n9; CE20:4n3; CE20:4n6; CE20:2n6; CE22:0; CE22:2n6; CE24:0; CESFA; PC20:4n6; PC22:5n6; PCn6; PCPUFA; PE20:4n6; TG15:0; TG18:2n6; TG20:0; TG20:2n6; TG20:4n6; TG20:5n3; TG22:0; TG22:2n6; TG22:1n9; TG22:4n6; TG22:5n6; TG24:1n9; TGn3; TGn6; TGPUFA; TL15:0; TL20:0; TL22:0; TL18:2n6; TL20:2n6; TL20:3n9; TL20:4n3; TL20:4n6; TL22:4n6; TL22:5n6; PC18:2n6; PC20:2n6; PE20:2n6; SM16:0; PGA2M; 6-keto-PGF1$\alpha$; 11-DTXB2; 12,13-DiHOME; 9,10-EpOME; 12,13-EpOME; PCLC; PC18:1n7; LY18:1n7; LY18:1n9; LY18:2n6; LY18:3n3; and 19,20-DiHDPA. In some embodiments, (a) the lipid metabolites PC18:3n6, PC20:3n6, CE14:0, CE16:1n7, CE18:1n9, CEMUFA, CEn7, CE18:1n7, CE18:2n6, CE18:3n6, CE22:5n3, CEn6, CEPUFA, PC14:0, PC16:1n7, PC18:1n9, PC18:3n3, PC18:4n3, PC20:0, PC20:1n9, PC20:4n3, PC20:5n3, PC22:0, PC22:1n9, PC24:0, PC24:1n9, PCdm, PCdm18:0, PCdm18:1n7, PCSFA, TG14:0, TG14:1n5, TG16:0, TG16:1n7, TG18:1n7, TGMUFA, TGn7, TGSFA, TL14:0, TL16:0, TL18:0, TL16:1n7, TL18:1n7, TL18:1n9, TL18:3n6, TL18:4n3, PC18:0, PC22:5n3, CE20:3n6, CELC, TGLC, TG18:3n6, TG20:4n3, TG20:3n6, TG22:5n3,LYLC, LY18:0, LY20:3n6, PE18:3n6, PE20:3n6, PE22:5n3, FA18:0, FA20:5n3, FA18:1n9, FA20:3n6, 15-HETE, and/or TL20:3n6 are positively associated with steatosis and/or NAFLD; and (b) the lipid metabolites CE14:1n5, CE18:0, CE20:0, CE20:1n9, CE20:3n9, CE20:4n3, CE20:4n6, CE20:2n6, CE22:0, CE22:2n6, CE24:0, CESFA, PC20:4n6, PC22:5n6, PCn6, PCPUFA, PE20:4n6, TG15:0, TG18:2n6, TG20:0, TG20:2n6, TG20:4n6, TG20:5n3, TG22:0, TG22:2n6, TG22:1n9, TG22:4n6, TG22:5n6, TG24:1n9, TGn3, TGn6, TGPUFA, TL15:0, TL20:0, TL22:0, TL18:2n6, TL20:2n6, TL20:3n9, TL20:4n3, TL20:4n6, TL22:4n6, TL22:5n6, PC18:2n6, PC20:2n6, PE20:2n6, SM16:0, PGA2M, 6-keto-PGF1$\alpha$, 11-DTXB2, 12,13-DiHOME, 9,10-EpOME, 12,13-EpOME, PCLC, PCl8:ln7, LY18:1n7, LY18:1n9, LY18:2n6, LY18:3n3, and/or 19,20-DiHDPA are negatively associated with steatosis and/or NAFLD. In some embodiments, the lipid metabolites that are measured comprise one or more fatty acid and the amount of each of the fatty acids is the relative amount of the fatty acid to total fatty acid content in the lipids of the lipid class (as indicated by the prefix preceding the fatty acid).

As used herein, metabolites that are "positively associated" or "positively correlated" with a disorder include those metabolites whose concentrations generally increase with the disorder relative to normal control subjects or a normal control reference. Metabolites that are "negatively associated" or "negatively correlated" with a disorder generally include those metabolites whose concentrations decrease with the disorder relative to normal control subjects or a normal control reference.

In some alternative embodiments, the liver disorder is NASH and the one or more lipid metabolites are selected from the group consisting of: PC18:3n6; PC20:3n6; CE14:0; CE16:ln7; CE18:1n9; CEMUFA; CEn7; CE18:1n7; CE18:2n6; CE18:3n6; CE22:5n3; CEn6; CEPUFA; PC14:0; PC16:1n7; PC18:1n9; PC18:3n3; PC18:4n3; PC20:0; PC20:1n9; PC20:4n3; PC20:5n3; PC22:0; PC22:1n9; PC24:0; PC24:1n9; PCdm; PCdm18:0; PCdm18:1n7; PCSFA; TG14:0; TG14:1n5; TG16:0; TG16:1n7; TG18:1n7; TGMUFA; TGn7; TGSFA; TL14:0; TL16:0; TL18:0; TH6:1n7; TL18:1n7; TL18:1n9; TL18:3n6; TL18:4n3; LY16:0; FA18:1n7; SM18:0; SM22:1n9; SMLC; PGB2; PGE2; PGF2$\alpha$; 15-keto-PGF2$\alpha$; 5-HETE; 8-HETE; 9-HETE; 11-HETE; 12-HETE; 12-HEPE; 11,12-EpETrE; 8,9-DiHETrE; PC18:0; PC22:5n3; CE20:3n6; CELC; TGLC; TG18:3n6; TG20:4n3; TG20:3n6; TG22:5n3;LYLC; LY18:0; LY20:3n6; PE18:3n6; PE20:3n6; PE22:5n3; FA18:0; FA20:5n3; FA18:1n9; FA20:3n6; 15-HETE; TL20:3n6; TG18:3n3; TG20:3n9; TG22:6n3; TG24:0; CE14:1n5; CE18:0; CE20:0; CE20:1n9; CE20:3n9; CE20:4n3; CE20:4n6; CE20:2n6; CE22:0; CE22:2n6; CE24:0; CESFA; PC20:4n6; PC22:5n6; PCn6; PCPUFA; PE20:4n6; TG15:0; TG18:2n6; TG20:0; TG20:2n6; TG20:4n6; TG20:5n3; TG22:0; TG22:2n6; TG22:1n9; TG22:4n6; TG22:5n6; TG24:1n9; TGn3; TGn6; TGPUFA; TL15:0; TL20:0; TL22:0; TL18:2n6; TL20:2n6; TL20:3n9; TL20:4n3; TL20:4n6; TL22:4n6; TL22:5n6; PC22:6n3; PE22:6n3; LY22:6n3;

PE14:0; PE18:1n7; PESFA; PELC; FA16:0; CE22:6n3; TL22:6n3; PCLC; PC18:1n7; LY18:1n7; LY18:1n9; LY18:2n6; LY18:3n3; and 19,20-DiHDPA. In some embodiments, (a) the lipid metabolites PC18:3n6, PC20:3n6, CE14:0, CE16:1n7, CE18:1n9, CEMUFA, CEn7, CE18:1n7, CE18:2n6, CE18:3n6, CE22:5n3, CEn6, CEPUFA, PC14:0, PC16:1n7, PC18:1n9, PC18:3n3, PC18:4n3, PC20:0, PC20:1n9, PC20:4n3, PC20:5n3, PC22:0, PC22:1n9, PC24:0, PC24:1n9, PCdm, PCdm18:0, PCdm18:1n7, PCSFA, TG14:0, TG14:1n5, TG16:0, TG16:1n7, TG18:1n7, TGMUFA, TGn7, TGSFA, TL14:0, TL16:0, TL18:0, TL16:1n7, TL18:1n7, TL18:1n9, TL18:3n6, TL18:4n3, LY16:0, FA18:1n7, SM18:0, SM22:1n9, SMLC, PGB2, PGE2, PGF2α, 15-keto-PGF2α, 5-HETE, 8-HETE, 9-HETE, 11-HETE, 12-HETE, 12-HEPE, 11,12-EpETrE, 8,9-DiHETrE, PC18:0, PC22:5n3, CE20:3n6, CELC, TGLC, TG18:3n6, TG20:4n3, TG20:3n6, TG22:5n3,LYLC, LY18:0, LY20:3n6, PE18:3n6, PE20:3n6, PE22:5n3, FA18:0, FA20:5n3, FA18:1n9, FA20:3n6, 15-HETE, and/or TL20:3n6 are positively associated with NASH; and (b) the lipid metabolites TG18:3n3, TG20:3n9, TG22:6n3, TG24:0, CE14:1n5, CE18:0, CE20:0, CE20:1n9, CE20:3n9, CE20:4n3, CE20:4n6, CE20:2n6, CE22:0, CE22:2n6, CE24:0, CESFA, PC20:4n6, PC22:5n6, PCn6, PCPUFA, PE20:4n6, TG15:0, TG18:2n6, TG20:0, TG20:2n6, TG20:4n6, TG20:5n3, TG22:0, TG22:2n6, TG22:1n9, TG22:4n6, TG22:5n6, TG24:1n9, TGn3, TGn6, TGPUFA, TL15:0, TL20:0, TL22:0, TL18:2n6, TL20:2n6, TL20:3n9, TL20:4n3, TL20:4n6, TL22:4n6, TL22:5n6, PC22:6n3, PE22:6n3, LY22:6n3, PE14:0, PE18:1n7, PESFA, PELC, FA16:0, CE22:6n3, TL22:6n3, PCLC, PC18:1n7, LY18:1n7, LY18:1n9, LY18:2n6, LY18:3n3, and/or 19,20-DiHDPA are negatively associated with NASH. In some embodiments, the lipid metabolites that are measured comprise one or more fatty acid and the amount of each of the fatty acids is the relative amount of the fatty acid to total fatty acid content in the lipids of the lipid class (as indicated by the prefix preceding the fatty acid).

It is understood that wherever embodiments are described herein with the language "comprising," otherwise analogous embodiments described in terms of "consisting of and/or "consisting essentially of are also provided.

"A", "an" and "the" include plural references unless the context clearly dictates otherwise.

Chemical terms, unless otherwise defined, are used as known in the art.

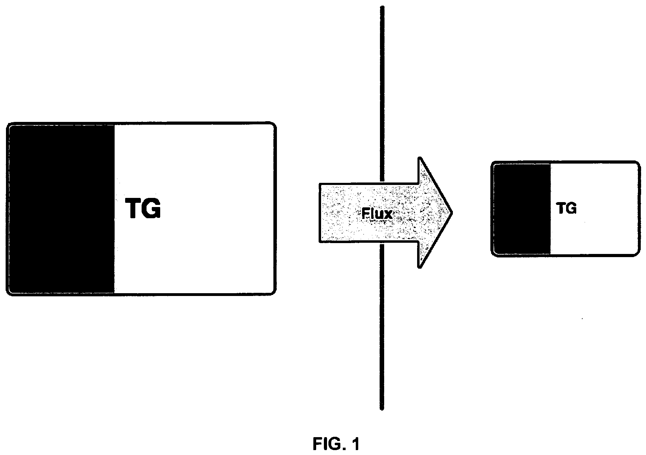

As shown in Figure 1, a lipid metabolite that is a relative proportion (shown in darker grey) of a triglyceride (or any other lipid class) can be measured in a body fluid, such as serum or plasma, as a quantitative measure of the relative proportion of that lipid metabolite in hepatic triglycerides (or other lipid class). If this relative proportion of lipid metabolite (or a collection of lipid metabolites) correlates with the hepatic triglyceride concentration, it serves as a quantitative surrogate of hepatic steatosis, independent of the flux of triglycerides from liver in VLDL. Thus, the mole percentage or other relative amount of a particular fatty acid within a particular lipid class may be used as a quantitative surrogate for steatosis.

In some embodiments, the relative amount (e.g., mole percentage or weight percent) of a single lipid metabolite may be used in the methods of the invention. In other embodiments, the relative amounts (e.g., mole percentages or weight percentages) of two or more lipid metabolites may be used in the methods of the invention, for example, 2, 3, 4, 5, 10, 15, 20, or more lipid metabolites. In some embodiments, the relative amount is the mole percentage. In some embodiments, the relative amount is the weight percentage. The amounts of one or more biomarkers, as defined below, in a sample from the subject may be used in the methods of the invention, in addition to the amount of one or more lipid metabolites. In some embodiments, the amount of the biomarker is the absolute amount of the biomarker in the sample. In some embodiments, the amount of the biomarker is the concentration of the biomarker in the sample.

According to the present invention, when analyzing the effects rendered by two or more lipid metabolites, one can either evaluate the effects of these lipid metabolites individually or obtain the net effect of these lipid metabolites, e.g., by using various mathematical formulas or models to quantify the effect of each lipid metabolite. A formula containing the levels of one or more lipid metabolites as variables includes any mathematical formula, model, equation, or expression established based on mathematic or statistical principles or methods using the values of one or more lipid metabolites as variables.

In general, any suitable mathematic analyses can be used to analyze the net effect of two or more lipid metabolites with respect to projecting the condition of the liver of a subject. For example, methods such as multivariate analysis of variance, multivariate regression, multiple regression can be used to determine relationships between dependent variables, and independent variables. Clustering, including both hierarchical and nonhierarchical methods, as well as nonmetric Dimensional Scaling can be used to determine associations among variables and among changes in those variables.

In addition, principle component analysis is a common way of reducing the dimension of studies, and can be used to interpret the variance-covariance structure of a data set. Principle components may be used in such applications as multiple regression and cluster analysis. Factor analysis is used to describe the covariance by constructing "hidden" variables from the observed variables. Factor analysis may be considered an extension of principle component analysis, where principle component analysis is used as parameter estimation along with the maximum likelihood method. Furthermore, simple hypothesis such as equality of two vectors of means can be tested using Hotelling's T squared statistic.

In some embodiments, a formula containing one or more lipid metabolites as variables is established by using regression analyses, e.g., multiple linear regressions. Examples of formulas developed include, without any limitation, the following:

$$\text{Formula I: } k + k_1(FA_1) + k_2(FA_2) + k_3(FA_3)$$

$$\text{Formula II: } k - k_1(FA_1) + k_2(FA_2) + k_3(FA_3)$$

$$\text{Formula III: } k + k_1(FA_1) - k_2(FA_2) + k_3(FA_3)$$

$$\text{Formula IV: } k + k_1(FA_1) + k_2(FA_2) - k_3(FA_3)$$

$$\text{Formula V: } k - k_1(FA_1) - k_2(FA_2) + k_3(FA_3)$$

$$\text{Formula VI: } k + k_1(FA_1) - k_2(FA_2) - k_3(FA_3)$$

$$\text{Formula VII: } k - k_1(FA_1) + k_2(FA_2) - k_3(FA_3)$$

$$\text{Formula VIII: } k - k_1(FA_1) - k_2(FA_2) - k_3(FA_3)$$

The formulas may use one or more lipid metabolites as variables, such as 1, 2, 3, 4, 5, 10, 15, 20, or more lipid metabolites. The constants of these formulas can be established by using a set of data obtained from known liver conditions. Usually the levels of lipid metabolites used in these formulas can be either the levels at a time point or changes of levels over a period of time.

According to the invention, mathematic formulas established using lipid metabolites can be used to either qualitatively or quantitatively assess the liver condition of a subject over a period of time. For example, a formula having one or more lipid metabolites as variables can be used to directly calculate the liver condition of a subject. In addition, the net value of a formula containing one or more lipid metabolites can be compared to the standard value of such formula corresponding to a liver condition pattern, e.g. progression or regression of fatty liver disease, and the results of such comparison can be used to project liver condition development. Specifically, a subject having a net value of a formula similar to or within the range of the standard value of such formula that is assigned to or associated with a progression of a liver condition is likely to experience a progression over a period of time. Similarly, a subject having a net value of a formula similar to or within the range of the standard values of such formula that is assigned to or associated with a regression is likely to experience a regression of their liver condition over a period of time.

Similarly, these mathematical modeling methods and formulas may also be used when analyzing the net effects rendered by one or more lipid metabolites and one or more biomarkers.

Lipid metabolites may be measured in a body fluid. Non-limiting examples of body fluids include, for example, fluids such as blood, plasma, serum, isolated lipoprotein fractions, saliva, urine, lymph, cerebrospinal fluid, and bile. In some embodiments, the lipid metabolite is measured in a blood-based body fluid, such as blood, plasma, serum, or lipoprotein fractions. In some embodiments, the lipid metabolite is measured in plasma. In some embodiments, the lipid metabolite is measured in serum.

In some embodiments, the invention provides methods in which the amounts of one or more, two or more, three or more, four or more, five or more, or six or more lipid metabolites are determined.

In some embodiments, the lipid metabolites which are measured comprise a pair of lipid metabolites selected from the group consisting of the one or more lipid metabolites comprise a pair of lipid metabolites selected from the group consisting of (a) 15-HETE and 15-keto-PGF2$\alpha$; (b) TG18:1n7 and PC20:3n6; (c) 11-HETE and CE22.6n3; (d) 11-HETE and PCTL; and (e) PC22:6n3 and PC18:3n3. In some embodiments, the method is a method of classifying a liver disorder as NASH versus NAFLD.

**Fatty Acid Markers for Steatosis, NAFLD, NASH, and/or Other Liver Disorders**

In some embodiments, the lipid metabolite is a fatty acid present within a particular lipid class. Lipid metabolites encompass, without limitation, each of the metabolites listed in Table 1 below, as well as each of the metabolites listed in Tables 7 and 8 of Example 4, below. In some embodiments, the lipid metabolite is TG20:4n6. The method may involve measuring the amount of more than one lipid metabolite, such as 2, 3, 4, 5, 10, 15, 20, or more lipid metabolites. In some embodiments, two or more lipid metabolites in Table 1 are measured. In some embodiments, three or more lipid metabolites in Table 1 are measured. In some embodiments, five or more lipid metabolites in Table 1 are measured. In some embodiments, two or more lipid metabolites in Tables 7 and/or 8 are measured. In some embodiments, three or more lipid metabolites in Tables 7 and/or 8 are measured. In some embodiments, five or more lipid metabolites in Tables 7 and/or 8 are measured. In some embodiments, the lipid metabolite is positively correlated with liver triglyceride levels. In some embodiments, the lipid metabolite is negatively correlated with liver triglyceride levels. In some embodiments, the lipid metabolite is measured as a relative amount within that particular lipid class. In some embodiments, the lipid metabolite is measured as a mole percentage within that particular lipid class. In some embodiments, the lipid metabolite is measured as a weight percentage within that particular lipid class.

Table 1. Blood-based Lipid Metabolite Markers of Hepatic Steatosis (Based on Mole Percentage)

| Lipid Class | Positive Correlates | Negative Correlates |
|---|---|---|
| **Triglycerides** | TG14:0 | TG15:0 |
| | TG14:1n5 | TG18:2n6 |
| | TG16:0 | TG18:3n3 |
| | TG18:1n7 | TG20:0 |
| | TGMUFA | TG20:2n6 |
| | TGn7 | TG20:3n6 |
| | TGSFA | TG20:3n9 |
| | TG16:1n7 | TG20:4n6 |
| | | TG20:5n3 |
| | | TG22:0 |
| | | TG22:1n9 |
| | | TG22:2n6 |
| | | TG22:4n6 |
| | | TG22:5n3 |
| | | TG22:5n6 |
| | | TG22:6n3 |
| | | TG24:0 |
| | | TG24:1n9 |
| | | TGn3 |
| | | TGn6 |
| | | TGPUFA |
| **Free Fatty Acids** | | FA16:1n7 |
| **Phospho-tidylcholines** | PC14:0 | PC18:1n7 |
| | PC16:1n7 | PC20:4n6 |
| | PC18:1n7 | PC22:5n6 |
| | PC18:1n9 | PCn6 |
| | PC18:3n3 | PCPUFA |
| | PC18:3n6 | PC22:5n3 |
| | PC 18:4n3 | |
| | PC20:0 | |

(continued)

| Lipid Class | Positive Correlates | Negative Correlates |
|---|---|---|
| | PC20: 1n9 | |
| | PC20:2n6 | |
| | PC20:3n6 | |
| | PC20:4n3 | |
| | PC20:5n3 | |
| | PC22:0 | |
| | PC22:1n9 | |
| | PC24:0 | |
| | PC24:1n9 | |
| | PCdm | |
| | PCdm 18:0 | |
| | PCdm18:1n7 | |
| | PCSFA | |
| Phospho-tidylethanol-amines | | PE20:4n6 |
| Cholesterol Esters | CE16:1n7 | CE14:1n5 |
| | CE18:1n7 | CE18:0 |
| | CE18:1n9 | CE20:0 |
| | CE18:2n6 | CE20:1n9 |
| | CE18:3n6 | CE20:2n6 |
| | CE22:5n3 | CE20:3n9 |
| | CE22:6n3 | CE20:4n3 |
| | CEMUFA | CE20:4n6 |
| | CEn6 | CE22:0 |
| | CEn7 | CE22:2n6 |
| | CEPUFA | CE24:0 |
| | CE14:0 | CESFA |
| Total Fatty Acids | 14:0 | 15:0 |
| | 16:0 | 20:0 |
| | 18:0 | 22:0 |
| | 16:1n7 | 18:2n6 |
| | 18:1n7 | 20:2n6 |
| | 18:1n9 | 20:3n9 |
| | 18:3n6 | 20:4n3 |
| | 18:4n3 | 20:4n6 |
| | | 22:4n6 |
| | | 22:5n6 |

In Table 1, the prefixes "TG", "FA", "PC", "PE", and "CE" correspond to fatty acids present within triglycerides, free fatty acids, phosphatidylcholines, phosphatidylethanolamines, and cholesterol esters, respectively. Thus, "TG14:0" indicates the fatty acid 14:0 present within triglycerides. In Table 1, "14:0" (without any prefix) indicates the fatty acid 14:0 present within total fatty acids.

The lipid class may be, for example, neutral lipids, phospholipids, free fatty acids, total fatty acids, triglycerides, cholesterol esters, phosphatidylcholines, phosphatidylethanolamines, diglycerides, or lysophosphatidylcholines. In some embodiments, the lipid class is selected from the group consisting of neutral lipids, phospholipids, free fatty acids, total fatty acids, triglycerides, cholesterol esters, phosphatidylcholines, and phosphatidylethanolamines. In some embodiments, the lipid class is selected from the group consisting of neutral lipids, phospholipids, total fatty acids, and cholesterol esters. In some embodiments, the lipid class is selected from the group consisting of free fatty acids, total fatty acids, triglycerides, cholesterol esters, phosphatidylcholines, and phosphatidylethanolamines. In some embodiments, the lipid class is free fatty acids. In some embodiments, the lipid class is total fatty acids. In some embodiments, the lipid class is triglycerides. In some embodiments, the lipid class is cholesterol esters. In some embodiments, the lipid class is phosphatidylcholines. In some embodiments, the lipid class is phosphatidylethanolamines. In some embodiments, the lipid class is phospholipids. In some embodiments, the lipid class is neutral lipids. In some embodiments, the lipid class is diglycerides. In some embodiments, the lipid class is sphingomyelins.

In some embodiments, one or more lipid metabolites are measured that comprise one or more fatty acids. In some embodiments, one or more lipid metabolites are selected from the group consisting of: PC18:3n6; PC20:3n6; CE14:0; CE16:ln7; CE18:1n9; CEMUFA; CEn7; CE18:1n7; CE18:2n6; CE18:3n6; CE22:5n3; CEn6; CEPUFA; PC14:0; PC16:1n7; PC18:1n9; PC18:3n3; PC18:4n3; PC20:0; PC20:1n9; PC20:4n3; PC20:5n3; PC22:0; PC22:1n9; PC24:0; PC24:1n9; PCdm; PCdm18:0; PCdm18:1n7; PCSFA; TG14:0; TG14:1n5; TG16:0; TG16:1n7; TG18:1n7; TGMUFA; TGn7; TGSFA; TL14:0; TL16:0; TL18:0; TL16:ln7; TL18:1n7; TL18:1n9; TL18:3n6; TL18:4n3; TG18:3n3; TG20:3n9; TG22:6n3; TG24:0; CE14:1n5; CE18:0; CE20:0; CE20:1n9; CE20:3n9; CE20:4n3; CE20:4n6; CE20:2n6; CE22:0; CE22:2n6; CE24:0; CESFA; PC20:4n6; PC22:5n6; PCn6; PCPUFA; PE20:4n6; TG15:0; TG18:2n6; TG20:0; TG20:2n6; TG20:4n6; TG20:5n3; TG22:0; TG22:2n6; TG22:1n9; TG22:4n6; TG22:5n6; TG24:1n9; TGn3; TGn6; TGPUFA; TL15:0; TL20:0; TL22:0; TL18:2n6; TL20:2n6; TL20:3n9; TL20:4n3; TL20:4n6; TL22:4n6; TL22:5n6; LY16:0; FA18:1n7; SM18:0; SM22:1n9; SMLC; PC18:0; PC22:5n3; CE20:3n6; CELC; TGLC; TG18:3n6; TG20:4n3; TG20:3n6; TG22:5n3; LYLC; LY18:0; LY20:3n6; PE18:3n6; PE20:3n6; PE22:5n3; FA18:0; FA20:5n3; FA18:1n9; FA20:3n6; TL20:3n6; PC18:2n6; PC20:2n6; PE20:2n6; SM16:0; PC22:6n3; PE22:6n3; LY22:6n3; PE14:0; PE18:1n7; PESFA; PELC; FA16:0; CE22:6n3; TL22:6n3; PCLC; PC18:1n7; LY18:1n7; LY18:1n9; LY18:2n6; and LY18:3n3. In some embodiments, the amount of each of the fatty acids is the relative amount of the fatty acid to total fatty acid content in the lipids of the lipid class (as indicated by the prefix preceding the fatty acid).

For instance, in some embodiments, one or more fatty acids are selected from the group consisting of: PC18:3n6; PC20:3n6; CE14:0; CE16:ln7; CE18:1n9; CEMUFA; CEn7; CE18:1n7; CE18:2n6; CE18:3n6; CE22:5n3; CEn6; CEPUFA; PC14:0; PC16:1n7; PC18:1n9; PC18:3n3; PC18:4n3; PC20:0; PC20:1n9; PC20:4n3; PC20:5n3; PC22:0; PC22:1n9; PC24:0; PC24:1n9; PCdm; PCdm18:0; PCdm18:1n7; PCSFA; TG14:0; TG14:1n5; TG16:0; TG16:1n7; TG18:1n7; TGMUFA; TGn7; TGSFA; TL14:0; TL16:0; TL18:0; TL16:1n7; TL18:1n7; TL18:1n9; TL18:3n6; TL18:4n3; TGl8:3n3; TG20:3n9; TG22:6n3; TG24:0; CE14:1n5; CE18:0; CE20:0; CE20:1n9; CE20:3n9; CE20:4n3; CE20:4n6; CE20:2n6; CE22:0; CE22:2n6; CE24:0; CESFA; PC20:4n6; PC22:5n6; PCn6; PCPUFA; PE20:4n6; TG15:0; TG18:2n6; TG20:0; TG20:2n6; TG20:4n6; TG20:5n3; TG22:0; TG22:2n6; TG22:1n9; TG22:4n6; TG22:5n6; TG24:1n9; TGn3; TGn6; TGPUFA; TL15:0; TL20:0; TL22:0; TL18:2n6; TL20:2n6; TL20:3n9; TL20:4n3; TL20:4n6; TL22:4n6; and TL22:5n6.

In some embodiments, the liver disorder is steatosis and/or NAFLD and one or more lipid metabolites are selected from the group consisting of: PC18:3n6; PC20:3n6; CE14:0; CE16:ln7; CE18:1n9; CEMUFA; CEn7; CE18:1n7; CE18:2n6; CE18:3n6; CE22:5n3; CEn6; CEPUFA; PC14:0; PC16:1n7; PC18:1n9; PC18:3n3; PC18:4n3; PC20:0; PC20:1n9; PC20:4n3; PC20:5n3; PC22:0; PC22:1n9; PC24:0; PC24:1n9; PCdm; PCdm18:0; PCdm18:1n7; PCSFA; TG14:0; TG14:1n5; TG16:0; TG16:1n7; TG18:1n7; TGMUFA; TGn7; TGSFA; TL14:0; TL16:0; TL18:0; TL16:ln7; TL18:1n7; TL18:1n9; TL18:3n6; TL18:4n3; PC18:0; PC22:5n3; CE20:3n6; CELC; TGLC; TG18:3n6; TG20:4n3; TG20:3n6; TG22:5n3; LYLC; LY18:0; LY20:3n6; PE18:3n6; PE20:3n6; PE22:5n3; FA18:0; FA20:5n3; FA18:1n9; FA20:3n6; TL20:3n6; CE14:1n5; CE18:0; CE20:0; CE20:1n9; CE20:3n9; CE20:4n3; CE20:4n6; CE20:2n6; CE22:0; CE22:2n6; CE24:0; CESFA; PC20:4n6; PC22:5n6; PCn6; PCPUFA; PE20:4n6; TG15:0; TG18:2n6; TG20:0; TG20:2n6; TG20:4n6; TG20:5n3; TG22:0; TG22:2n6; TG22:1n9; TG22:4n6; TG22:5n6; TG24:1n9; TGn3; TGn6; TGPUFA; TL15:0; TL20:0; TL22:0; TL18:2n6; TL20:2n6; TL20:3n9; TL20:4n3; TL20:4n6; TL22:4n6; TL22:5n6; PC18:2n6; PC20:2n6; PE20:2n6; SM16:0; PCLC; PC18:1n7; LY18:1n7; LY18:1n9; LY18:2n6; and LY18:3n3.

In some embodiments, the lipid metabolites PC18:3n6, PC20:3n6, CE14:0, CE16:1n7, CE18:1n9, CEMUFA, CEn7, CE18:1n7, CE18:2n6, CE18:3n6, CE22:5n3, CEn6, CEPUFA, PC14:0, PC16:1n7, PC18:1n9, PC18:3n3, PC18:4n3, PC20:0, PC20:1n9, PC20:4n3, PC20:5n3, PC22:0, PC22:1n9, PC24:0, PC24:1n9, PCdm, PCdm 18:0, PCdm18:1n7, PCSFA, TG14:0, TG14:1n5, TG16:0, TG16:1n7, TG18:1n7, TGMUFA, TGn7, TGSFA, TL14:0, TL16:0, TL18:0, TL16:1n7, TL18:1n7, TL18:1n9, TL18:3n6, TL18:4n3, PC18:0, PC22:5n3, CE20:3n6, CELC, TGLC, TG18:3n6,

TG20:4n3, TG20:3n6, TG22:5n3,LYLC, LY18:0, LY20:3n6, PE18:3n6, PE20:3n6, PE22:5n3, FA18:0, FA20:5n3, FA18:1n9, and/or FA20:3n6 are positively associated with steatosis and/or NAFLD. 1n some embodiments, the lipid metabolites CE14:1n5, CE18:0, CE20:0, CE20:1n9, CE20:3n9, CE20:4n3, CE20:4n6, CE20:2n6, CE22:0, CE22:2n6, CE24:0, CESFA, PC20:4n6, PC22:5n6, PCn6, PCPUFA, PE20:4n6, TG15:0, TG18:2n6, TG20:0, TG20:2n6, TG20:4n6, TG20:5n3, TG22:0, TG22:2n6, TG22:1n9, TG22:4n6, TG22:5n6, TG24:1n9, TGn3, TGn6, TGPUFA, TL15:0, TL20:0, TL22:0, TL18:2n6, TL20:2n6, TL20:3n9, TL20:4n3, TL20:4n6, TL22:4n6, TL22:5n6, PC18:2n6, PC20:2n6, PE20:2n6, SM16:0, PCLC, PC18:1n7, LY18:1n7, LY18:1n9, LY18:2n6, and/or LY18:3n3 are negatively associated with steatosis and/or NAFLD.

In some alternative embodiments, the liver disorder is NASH and one or more lipid metabolites are selected from the group consisting of: PC18:3n6; PC20:3n6; CE14:0; CE16:1n7; CE18:1n9; CEMUFA; CEn7; CE18:1n7; CE18:2n6; CE18:3n6; CE22:5n3; CEn6; CEPUFA; PC14:0; PC16:1n7; PC18:1n9; PC18:3n3; PC18:4n3; PC20:0; PC20:1n9; PC20:4n3; PC20:5n3; PC22:0; PC22:1n9; PC24:0; PC24:1n9; PCdm; PCdm 18:0; PCdm18:1n7; PCSFA; TG14:0; TG14:1n5; TG16:0; TG16:1n7; TG18:1n7; TGMUFA; TGn7; TGSFA; TL14:0; TL16:0; TL18:0; TL16:ln7; TL18:1n7; TL18:1n9; TL18:3n6; TL18:4n3; LY16:0; FA18:1n7; SM18:0; SM22:1n9; SMLC; PC18:0; PC22:5n3; CE20:3n6; CELC; TGLC; TG18:3n6; TG20:4n3; TG20:3n6; TG22:5n3;LYLC; LY18:0; LY20:3n6; PE18:3n6; PE20:3n6; PE22:5n3; FA18:0; FA20:5n3; FA18:1n9; FA20:3n6; 15-HETE; TL20:3n6; TG18:3n3; TG20:3n9; TG22:6n3; TG24:0; CE14:1n5; CE18:0; CE20:0; CE20:1n9; CE20:3n9; CE20:4n3; CE20:4n6; CE20:2n6; CE22:0; CE22:2n6; CE24:0; CESFA; PC20:4n6; PC22:5n6; PCn6; PCPUFA; PE20:4n6; TG15:0; TG18:2n6; TG20:0; TG20:2n6; TG20:4n6; TG20:5n3; TG22:0; TG22:2n6; TG22:1n9; TG22:4n6; TG22:5n6; TG24:1n9; TGn3; TGn6; TGPUFA; TL15:0; TL20:0; TL22:0; TL18:2n6; TL20:2n6; TL20:3n9; TL20:4n3; TL20:4n6; TL22:4n6; TL22:5n6; PC22:6n3; PE22:6n3; LY22:6n3; PE14:0; PEI8:1n7; PESFA; PELC; FA16:0; CE22:6n3, TL22:6n3; PCLC; PC18:1n7; LY18:1n7; LY18:1n9; LY18:2n6; and LY18:3n3.

In some embodiments, the lipid metabolites PC18:3n6, PC20:3n6, CE14:0, CE16:1n7, CE18:1n9, CEMUFA, CEn7, CE18:1n7, CE18:2n6, CE18:3n6, CE22:5n3, CEn6, CEPUFA, PC14:0, PC16:1n7, PC18:1n9, PC18:3n3, PC18:4n3, PC20:0, PC20: 1n9, PC20:4n3, PC20:5n3, PC22:0, PC22:1n9, PC24:0, PC24:1n9, PCdm, PCdm18:0, PCdm18:1n7, PCSFA, TG14:0, TG14:1n5, TG16:0, TG16:1n7, TG18:1n7, TGMUFA, TGn7, TGSFA, TL14:0, TL16:0, TL18:0, TL16:1n7, TL18:1n7, TL18:1n9, TL18:3n6, TL18:4n3, LY16:0, FA18:1n7, SM18:0, SM22:1n9, SMLC, PC18:0, PC22:5n3, CE20:3n6, CELC, TGLC, TG18:3n6, TG20:4n3, TG20:3n6, TG22:5n3,LYLC, LY18:0, LY20:3n6, PE18:3n6, PE20:3n6, PE22:5n3, FA18:0, FA20:5n3, FA18:1n9, FA20:3n6, and/or TL20:3n6 are positively associated with NASH. In some embodiments, the lipid metabolites TG18:3n3, TG20:3n9, TG22:6n3, TG24:0, CE14:1n5, CE18:0, CE20:0, CE20:1n9, CE20:3n9, CE20:4n3, CE20:4n6, CE20:2n6, CE22:0, CE22:2n6, CE24:0, CESFA, PC20:4n6, PC22:5n6, PCn6, PCPUFA, PE20:4n6, TG15:0, TG18:2n6, TG20:0, TG20:2n6, TG20:4n6, TG20:5n3, TG22:0, TG22:2n6, TG22:ln9, TG22:4n6, TG22:5n6, TG24:1n9, TGn3, TGn6, TGPUFA, TL15:0, TL20:0, TL22:0, TL18:2n6, TL20:2n6, TL20:3n9, TL20:4n3, TL20:4n6, TL22:4n6, TL22:5n6, PC22:6n3, PE22:6n3, LY22:6n3, PE14:0, PE18:1n7, PESFA, PELC, FA16:0, CE22:6n3, TL22:6n3, PCLC, PC18:1n7, LY18:1n7, LY18:1n9, LY18:2n6, and/or LY18:3n3 are negatively associated with NASH.

In some embodiments, if the relative amount of PC18:3n6, PC20:3n6, CE14:0, CE16:1n7, CE18:1n9, CEMUFA, CEn7, CE18:1n7, CE18:2n6, CE18:3n6, CE22:5n3, CEn6, CEPUFA, PC14:0, PC16:1n7, PC18:1n9, PC18:3n3, PC18:4n3, PC20:0, PC20:1n9, PC20:4n3, PC20:5n3, PC22:0, PC22:1n9, PC24:0, PC24:1n9, PCdm, PCdm18:0, PCdm 18:1n7, PCSFA, TG14:0, TG14:1n5, TG16:0, TG16:1n7, TG18:1n7, TGMUFA, TGn7, TGSFA, TL14:0, TL16:0, TL18:0, TL16:1n7, TL18:1n7, TL18:1n9, TL18:3n6, and/or TL18:4n3 is greater than a reference (e.g., a normal control), then accumulation of triglycerides in the liver is indicated. In some embodiments, hepatic impairment, hepatic steatosis, NAFLD, and/or NASH is indicated.

In some embodiments, if the relative amount of CE14:1n5, CE18:0, CE20:0, CE20:1n9, CE20:3n9, CE20:4n3, CE20:4n6, CE20:2n6, CE22:0, CE22:2n6, CE24:0, CESFA, PC20:4n6, PC22:5n6, PCn6, PCPUFA, PE20:4n6, TG15:0, TG18:2n6, TG20:0, TG20:2n6, TG20:4n6, TG20:5n3, TG22:0, TG22:2n6, TG22:1n9, TG22:4n6, TG22:5n6, TG24:1n9, TGn3, TGn6, TGPUFA, TL15:0, TL20:0, TL22:0, TL18:2n6, TL20:2n6, TL20:3n9, TL20:4n3, TL20:4n6, TL22:4n6, and/or TL22:5n6 is lower than a reference (e.g., a normal control), then accumulation of triglycerides in the liver is indicated. In some embodiments, hepatic impairment, hepatic steatosis, NAFLD, and/or NASH is indicated.

In some embodiments, the amounts of the fatty acids (e.g., the relative amounts of the fatty acids within particular lipid classes) are determined from a blood, serum, plasma, or isolated lipoprotein fraction sample.

### Eicosanoid Markers for Steatosis, NAFLD, NASH, and/or Other Liver Disorders

The present invention provides methods in which one, some, or all of the lipid metabolites measured in the sample(s) may be eicosanoids. Non-limiting, exemplary eicosanoids are provided in Table 2, in Table 9 in Example 5, and in Table 10 in Example 5. Exemplary abbreviations for eicosanoids are indicated in Table 9.

Table 2. List of Eicosanoids

| | | |
|---|---|---|
| 13-14-dihydro-15-keto PGA2 | PGB2 | PGD2 |
| PGE2 | 6-keto PGF 1a | PGF2a |
| 11b-PGF2a | 15-keto PGF2a | PGJ2 |
| 15-deoxy-o-12,14-PGJ2 | TXB2 | 11-dehvdro TXB2 |
| 8-iso-PGF2a | 9-HODE | 13-HODE |
| 5-HETE | 8-HETE | 9-HETE |
| 11-HETE | 12-HETE | 15-HETE |
| 5(S)-HEPE | 12(S)-HEPE | 15(S)-HEPE |
| LTB4 | LTB5 | LTC4 |
| LTD4 | LTE4 | LTF4 |
| Lipoxin A4 | 20-HETE | 12(13)-DiHOME |
| 12(13)-EpOME | 9(10)-EpOME | 5(6)-EpETrE |
| 11(12)-EpETrE | 14(15)-EpETrE | 5,6-DiHETrE |
| 8,9-DiHETrE | 11,12-DiHETrE | 14,15-DiHETrE |
| 14,15-DiHETE | 17,18-DiHETE | 14(15)-EpETE |
| 17(18)-EpETE | 19(20)-DiHDPA | |
| 6kPGF1a | PGJ2 | 8,9 DiHETrE |
| D8-12 HETE | D4-6 keto PGF 1a | PGB2 |
| 5,6 DiHETrE | 9 HETE | d4-8-iso-PGF2a |
| LTB5 | 20 HETE | 11(12) EpETrE |
| D4-PGF2a | D4- PGB2 | 15 HEPE |
| 11 HETE | 11 bPGF2a | LTC4 |
| 15 deoxy 12,14 PGJ2 | 8 HETE | TXB2 |
| LTE4 | 12 (S) HEPE | 14(15) EpETE |
| D4- TXB2 | LTF4 | 5 (S) HEPE |
| 12 HETE | 8-iso-PGF2a | 13,14 dihydro 15 keto PGA2 |
| D4- 13 HODE | D8-5 HETE | PGF2a |
| LTD4 | D4- 9 HODE | 5 HETE |
| D4- PGE2 | 17,18 DiHETE | 13 HODE |
| 5(6) EpETrE | D4- PGD2 | D4- LTB4 |
| 12(13) EpOME | 11 dehvdro TXB2 | LTB4 |
| 9 HODE | D4-11 dhTXB2 | 14,15 DiHETE |
| 9(10) EpOME | PGE2 | 12(13) DiHOME |
| D8-15 HETE | PGD2 | 14,15 DiHETrE |
| 15 HETE | 15 keto PGF2a | 19,20 DiHDPA |
| 14(15) EpETrE | Lipoxin A4 | 11,12 DiHETrE |
| 17(18) EpETE | | |

In some embodiments, the method may involve measuring the amount of more than one lipid metabolite, such as 2, 3, 4, 5, 10, 15, 20, or more lipid metabolites, which may include 2, 3, 4, 5, 10, 15, 20, or more fatty acid markers described herein and/or 2, 3, 4, 5, 10, 15, 20, or more eicosanoid markers described herein. In some embodiments, two or more lipid metabolites in Table 2 are measured. In some embodiments, three or more lipid metabolites in Table 2 are measured. In some embodiments, five or more lipid metabolites in Table 2 are measured. In some embodiments, two or more lipid metabolites in Tables 9 and/or 10 (see Example 5, below) are measured. In some embodiments, three or more lipid metabolites in Tables 9 and/or 10 are measured. In some embodiments, five or more lipid metabolites in Tables 9 and/or 10 are measured. In some embodiments, two or more lipid metabolites in Table 1, Table 2, Table 7 (see Example 4, below), Table 8 (see Example 4, below), Table 9, and/or Table 10 are measured. In some embodiments, three or more lipid metabolites in Table 1, Table 2, Table 7, Table 8, Table 9, and/or Table 10 are measured. In some embodiments, five or more lipid metabolites in Table 1, Table 2, Table 7, Table 8, Table 9, and/or Table 10 are measured. In some embodiments, two or more lipid metabolites in Table 7, Table 8, and/or Table 10 are measured. In some embodiments, three or more lipid metabolites in Table 7, Table 8, and/or Table 10 are measured. In some embodiments, five or more lipid metabolites in Table 7, Table 8, and/or Table 10 are measured.

In some embodiments, one or more lipid metabolites are selected from the group consisting of PGB2; PGE2; PGF2$\alpha$; 15-keto-PGF2$\alpha$; 5-HETE; 8-HETE; 9-HETE; 11-HETE; 12-HETE; 12-HEPE; 11, 12-EpETrE; 8,9-DiHETrE. 15-HETE; PGA2M; 6-keto-PGF1$\alpha$; 11-DTXB2; 12,13-DiHOME; 9, 10-EpOME; 12, 13-EpOME; and 19,20-DiHDPA.

In some embodiments, the following eicosanoids are positively associated with NASH : PGB2; PGE2; PGF2$\alpha$; 15-keto-PGF2$\alpha$; 5-HETE; 8-HETE; 9-HETE; 11-HETE; 12-HETE; 12-HEPE; 11, 12-EpETrE; 8,9-DiHETrE; and 15-HETE. In some embodiments, 15-HETE is positively associated with steatosis and/or NAFLD. In some embodiments, the following eicosanoids are negatively associated with steatosis and/or NAFLD: PGA2M; 6- keto-PGF1$\alpha$; 11-DTXB2; 12,13-DiHOME; 9, 10-EpOME; 12, 13-EpOME; and 19, 20-DiHDPA. In some embodiments, the eicosanoid 19, 20-DiHDPA is negatively associated with NASH.

In certain embodiments, the method is a method of diagnosing NASH in a subject, comprising not only determining a relative amount of one or more fatty acids to total fatty acid content in the lipids of one or more lipid classes in a sample from a body fluid of the subject, but also the step of determining the level of an eicosanoid in a body fluid from the subject. In some embodiments, a higher than normal level of the eicosanoid is indicative of NASH. In some embodiments, the eicosanoid is selected from the group consisting of 15-HETE; PGB2; PGE2; PGF2$\alpha$; 15-keto-PGF2$\alpha$; 5-HETE; 8-HETE; 9-HETE; 11-HETE; 12- HETE; 12-HEPE; 11,12-EpETrE; and 8,9-DiHETrE.

In some embodiments, the amounts of the eicosanoids are determined from a blood, serum, plasma, or isolated lipoprotein fraction sample.

Other Biomarkers for Steatosis, NAFLD, NASH, and/or Other Liver Disorders

The invention further provides, in some embodiments, methods in which not only the amount of one or more lipid metabolites are determined in a sample, but also the amount of one or more additional biomarkers is determined.

The following additional biomarkers may aid the diagnosis of steatosis, NAFLD and NASH:

(1) malonyl-CoA and malonylcarnitine (levels increase with increasing levels of triglycerides in liver);

(2) free carnitine, butyrobetaine, and acylcarnitines listed in Table 3 (levels decrease with increasing levels of triglycerides in liver) or in Example 6; and/or

(3) the sterols and bile acids listed in Table 4 (levels increase with increased cholesterol synthesis) or in Example 6.

Body fluid and cellular samples may be used to measure these additional biomarkers. Examples of cellular samples include, but are not limited to, lymphocytes and macrophages.

Table 3. List of Acylcarnitines

| | | |
|---|---|---|
| L-Carnitine | Butyrobetaine | Acetyl carnitine |
| Propionyl carnitine | Butyryl carnitine | Hexanoyl carnitine |
| Valeryl carnitine | Octanoyl carnitine | Decanoyl carnitine |
| Myristoyl carnitine | Palmitoyl carnitine | Stearoyl carnitine |
| Oleoyl carnitine | Linoleovl carnitine | Arachidoyl carnitine |
| Dodecanoyl carnitine | | |

Table 4. List of Bile Acids and Sterols

| Cholic Acid | Chenodeoxycholic Acid | Deoxycholic Acid |
|---|---|---|
| Lithocholic Acid | Glycocholic Acid | Taurodeoxycholate |
| Glycochenodeoxycholate | Taurochenodeoxycholate | β-Muricholic Acid |
| Taurolithocholic acid | Ursodeoxycholic acid | Taurodeoxycholic acid |
| Taurocholic acid | Glycodesoxycholic acid | Glycolithocholic acid |
| Glycoursodeoxycholic acid | Cholesterol | Coprostanol |
| Cholestanol | Lanosterol | Lathosterol |
| β-Sitosterol | Desmosterol | Campesterol |
| Coprosterol | Lathosterol | Campesterol |
| Stigmasterol | 4-Cholesten-3-One | Fucosterol |

Additionally, the following additional biomarkers may aid in the diagnosis of NASH as distinct from NAFLD:

(1) The sterols and bile acids listed in Table 4 (levels increase with increased cholesterol synthesis) or in Example 6;

(2) Eicosanoids including, but not limited to, those shown in Table 2 (above), in Table 9 of Example 5, or in Table 10 of Example 5; and/or

(3) Cytokines, cytokeratine, chemokines, adipokines or leptins including, but not limited to, TNF$\alpha$, IL-6, CCL2/MCP-1 and CCL19 (level increase in NASH); IL-8, IL-18, cytokeratine 8 and cytokeratine 18 (levels decrease inNASH).

Body fluid and cellular samples may be used to measure the additional markers. Examples of cellular samples include, but are not limited to, lymphocytes and macrophages.

Further information on these biomarkers may be found in: (cytokines) Haukeland JW, et al. Systemic inflammation in nonalcoholic fatty liver disease is characterized by elevated levels of CCL2. J Hepatol. 2006 Jun;44(6):1167-74; and Abiru S, et al. Serum cytokine and soluble cytokine receptor levels in patients with non-alcoholic steatohepatitis. Liver Int. 2006 Feb;26(1):3945; (malonyl-CoA) Savage DB, et al. Reversal of diet-induced hepatic steatosis and hepatic insulin resistance by antisense oligonucleotide inhibitors of acetyl-CoA carboxylases 1 and 2. J Clin Invest. 2006 Mar;116(3):817-24; and Hammond LE, et al. Mitochondrial glycerol-3-phosphate acyltransferase-1 is essential in liver for the metabolism of excess acyl-CoAs. J Biol Chem. 2005 Jul 8;280(27):25629-36; (butyrobetaine) Higashi Y, et al. Effect of gamma-butyrobetaine on fatty liver in juvenile visceral steatosis mice. J Pharm Pharmacol. 2001 Apr;53(4):527-33.

Measurements of the amounts of one or more of these additional biomarkers may be used in the methods of the invention, in addition to measurement of a lipid metabolite. In some embodiments, the amount of one of the biomarkers is measured in a sample from the subject. In some embodiments, the amounts of two of the biomarkers are measured in a sample from the subject. In other embodiments, 3, 4, 5, 6, 7, 8, 10, 12, 15, 20, or more of the biomarkers may be measured in a sample from the subject.

**Methods of Diagnosing and Monitoring**

The methods of the invention may be used to diagnose NASH. The methods may also be used to assess the severity of a liver disorder, monitor a liver disorder, and/or assess the progression or regression of a liver disorder.

In some embodiments, the methods comprise comparing the amounts(s) of one or more lipid metabolites to one or more references. In some embodiments, a reference represents the normal level of the lipid metabolite. In some embodiments, a reference is an amount of the lipid metabolite previously measure for the same subject. In some embodiments, the reference is a relative amount of the one or more fatty acids to total fatty acid content in the triglycerides in a sample from a body fluid previously obtained from the subject. In some embodiments, the reference represents the relative amount of the one or more fatty acids to total fatty acid content in the triglycerides found in one or more samples from a body fluid of one or more subjects having normal livers.

For example, a method of diagnosis may comprise determining a relative amount of one or more fatty acids to total fatty acid content in the lipids of one or more lipid classes in a sample from a body fluid of the subject, and correlating that amount with the presence of the liver disorder. In some embodiments, the method may further comprise the step of comparing the relative amount to a reference, wherein if the relative amount is greater than the reference, hepatic impairment, hepatic steatosis, NAFLD, steatohepatitis, or NASH is indicated. In some embodiments, the method may further comprise the step of comparing the relative amount to a reference, wherein if the relative amount is less than the reference, hepatic impairment, hepatic steatosis, NAFLD, steatohepatitis, or NASH is indicated.

Similarly, the severity of the liver disorder may be measured, wherein the relative amount indicates the severity of the liver disorder. Additionally, the relative amount indicates the current state of the liver, and thus a liver disorder may be monitored and/or the progression or regression of the disorder assessed. The relative amount may be measured at two or more time points. In some embodiments, the relative amount may be measured at 2, 3, 4, 5, 6, 7, 8, 10, 12, 15, 20, or more time points. Each time point may be separated by one or more hours, days, weeks, or months. By measuring the relative amount at more than one time point, the clinician may assess a subject's response to treatment.

In some embodiments, the relative amount may be compared to a reference. In some embodiments, if the relative amount is greater than the reference, hepatic impairment, hepatic steatosis, NAFLD, steatohepatitis, or NASH is indicated. In some embodiments, if the relative amount is less than the reference, hepatic impairment, hepatic steatosis, NAFLD, steatohepatitis, or NASH is indicated. The difference between the relative amount and the reference may also be used to indicate severity. For example, as the relative amount becomes increasingly greater than the reference, increasing severity of disease is indicated. Or, for example, as the relative amount becomes increasingly less than the reference, increasing severity of disease is indicated. Exemplary references may be based on the amount(s) of a lipid metabolite(s) from, but not limited to, individuals with normal livers, individuals with hepatic impairment, individuals with steatosis, individuals with NAFLD, individuals with steatohepatitis, individuals with NASH, individuals with cirrhosis, and/or individuals with fibrosis. The reference may also be based on individuals with a liver disorder resulting from a particular cause, for example, one or more of those found below. The reference may also be based on samples previously obtained from the subject, for example, before the liver disorder developed, before treatment began, after treatment was ended, and/or at different time points during treatment. In some embodiments, the reference is the relative amount of one or more fatty acids to total fatty acid content in one or more lipid classes in one or more samples of a body fluid previously obtained from the subject. In some embodiments, the reference represents the relative amount of one or more fatty acids to total fatty acid content in one or more lipid classes in one or more samples of a body fluid of one or more subjects having normal livers.

In some embodiments, the subject is a mammal. In some embodiments, the mammal is a primate. In some embodiments, the subject is a human.

In some embodiments, the method is a method of monitoring a liver disorder that is used to determine the subject's response to treatment.

Causes of Steatosis, NAFLD, Steatohepatitis and NASH

The fatty acid liver disorders that may benefit from the methods disclosed herein may be caused by a variety of factors. Non-limiting examples include: hepatitis; steatosis induced by viral or non-viral infectious agents, such as yellow fever, HIV, HBV, and HCV; drug-induced steatosis, such as by tamoxifen, uncoupling protein inhibitors, Isoniazid, Rifampicin, fibrates, and peroxisome proliferator-activated receptor (PPAR) agonists; metabolic causes, such as obesity, polycystic ovary syndrome (PCOS), diabetes, insulin resistance, and metabolic disorder; alcohol-based causes such as alcoholic fatty liver disease and alcoholic steatohepatitis; inborn errors of metabolism or genetic alterations, such as citrin deficiency, hemochromatosis, and hyperferritinemia; toxin-induced causes, such as toxin- induced steatosis or toxin-induced steatohepatitis, for example, by carbon tetrachloride;
malnutrition; impaired nutrient absorption; celiac disease; lipodystrophy; bariatric surgery; and liver transplants.

Thus, in some embodiments, the liver disorder is associated with one or more conditions selected from the group consisting of: hepatitis, HIV infection, HBV infection, HCV infection, viral-induced steatosis, steatosis induced by a non-viral infectious agent, drug-induced steatosis, obesity, polycystic ovary syndrome (PCOS), diabetes, insulin resistance, metabolic disorder, alcoholic fatty liver disease, alcoholic steatohepatitis, an inborn error of metabolism, a genetic alteration, toxin-induced steatosis, toxin-induced steatohepatitis, malnutrition, impaired nutrient absorption, celiac disease, lipodystrophy, bariatric surgery, and a liver transplant.

The diagnostic methods may also be used for the assessment of liver grafts, suitability of individuals for liver graft donation, evaluation before bariatric surgery, evaluation of bariatric surgery patients to assess response to surgery, and evaluation of weight loss patients.

**Methods of Measurement of Lipid Metabolites and Biomarkers**

Assays for lipid metabolite content may be performed on a body fluid sample. In some embodiments, the amounts of the lipid metabolites are determined from sample(s) selected from the group consisting of blood, plasma, serum, isolated lipoprotein fraction, saliva, urine, lymph fluid, and cerebrospinal fluid. In some embodiments, the assays may be performed on whole blood, plasma, serum, or isolated lipoprotein fractions. In some embodiments, the sample(s) are plasma or serum. Assays for the additional biomarkers may be performed on a body fluid or a cellular sample.

In some embodiments, multiple different lipid metabolites are measured in the same sample. In other embodiments, each of multiple lipid metabolites are measured from a different sample. If multiple samples are used, the samples may be from the same or different body fluids of the subject.

The lipid metabolites and other biomarkers may readily be isolated and/or quantified by methods known to those of skill in the art, including, but not limited to, methods utilizing: mass spectrometry (MS), high performance liquid chromatography (HPLC), isocratic HPLC, gradient HPLC, normal phase chromatography, reverse phase HPLC, size exclusion chromatography, ion exchange chromatography, capillary electrophoresis, microfluidics, chromatography, gas chromatography (GC), thin-layer chromatography (TLC), immobilized metal ion affinity chromatography (IMAC), affinity chromatography, immunoassays, and/or colorimetric assays. In some embodiments, the methods of the invention utilize MS to determine lipid metabolite content. In some embodiments, the methods of the invention utilize an immunoassay to determine lipid metabolite content. In some embodiments, the methods of the invention utilize MS to determine the concentration of a biomarker. In some embodiments, the methods of the invention utilize an immunoassay to determine the concentration of a biomarker.

Various analytical methods are well known to those of skill in the art, and are further described in the following documents: MS: Cyr D, et al. A GC/MS validated method for the nanomolar range determination of succinylacetone in amniotic fluid and plasma: an analytical tool for tyrosinemia type I. J Chromatogr B Analyt Technol Biomed Life Sci. 2006Feb 17;832(1):24-9; Vogeser M. Abstract Liquid chromatography-tandem mass spectrometry-application in the clinical laboratory. Clin Chem Lab Med. 2003 Feb; 41(2):117-26. HPLC: Khalil PN, et al. Validation and application of a high-performance liquid chromatographic-based assay for determination of the inosine 5'-monophosphate dehydrogenase activity in erythrocytes. J Chromatogr B Analyt Technol Biomed Life Sci. 2006 May 23; Fouassier M, et al. Determination of serotonin release from platelets by HPLC and ELISA in the diagnosis of heparin-induced thrombocytopenia: comparison with reference method by [C]-serotonin release assay; J Thromb Haemost. 2006 May; 4(5):1136-9; Badiou S, et al. Determination of plasma amino acids by fluorescent derivatization and reversed-phase liquid chromatographic separation. Clin Lab. 2004; 50(3-4): 153-8; Brunelli T, et al. Comparison of three methods for total homocysteine plasma determination. Clin Lab. 2001; 47(7-8):393-7. CE: Zinellu A, et al. Assay for the simultaneous determination of guanidinoacetic acid, creatinine and creatine in plasma and urine by capillary electrophoresis UV-detection. J Sep Sci. 2006 Mar; 29(5):704- 8; Jabeen R, et al. Capillary electrophoresis and the clinical laboratory. Electrophoresis. 2006 May 23; Gao P, et al. Rapid detection of Staphylococcus aureus by a combination of monoclonal antibody-coated latex and capillary electrophoresis. Electrophoresis. 2006 May; 27(9): 1784-9. Microfluidics: Johannessen EA, et al. A suspended membrane nanocalorimeter for ultralow volume bioanalysis. IEEE Trans Nanobioscience. 2002 Mar; 1 (1):29-36; Herrmann M, et al. Enzymatically-generated fluorescent detection in microchannels with internal magnetic mixing for the development of parallel microfluidic ELISA; Lab Chip. 2006 Apr;6(4):555-60. Epub 2006 Mar 3; Yang S, et al. Blood plasma separation in microfluidic channels using flow rate control. ASAIO J. 2005 Sep-Oct;51(5):585-90; Dupuy AM, et al. Protein biochip systems for the clinical laboratory; Clin Chem Lab Med. 2005;43(12):1291-302. Chromatography: Paterson S, et al. Validation of techniques to detect illicit heroin use in patients prescribed pharmaceutical heroin for the management of opioid dependence. Addiction. 2005 Dec;100(12): 1832-9; Bottcher M, et al. Evaluation of buprenorphine CEDIA assay versus GC-MS and ELISA using urine samples from patients in substitution treatment. J Anal Toxicol. 2005 Nov-Dec;29(8):769-76; Julak J. Chromatographic analysis in bacteriologic diagnostics of blood cultures, exudates, and bronchoalveolar lavages. Prague Med Rep. 2005;106(2):175-94; Boettcher M, et al. Precision and comparability of Abuscreen OnLine assays for drugs of abuse screening in urine on Hitachi 917 with other immunochemical tests and with GC/MS. Clin Lab. 2000;46(1-2):49-52. Immunoassays: Boettcher M, et al. Precision and comparability of Abuscreen OnLine assays for drugs of abuse screening in urine on Hitachi 917 with other immunochemical tests and with GC/MS. Clin Lab. 2000;46(1-2):49-52; Westermann J, et al. Simple, rapid and sensitive determination of epinephrine and norepinephrine in urine and plasma by non-competitive enzyme immunoassay, compared with HPLC method. Clin Lab. 2002;48(1-2):61-71; Aoyagi K, et al. Performance of a conventional enzyme immunoassay for hepatitis C virus core antigen in the early phases of hepatitis C infection. Clin Lab. 2001;47(3-4):119-27; Hubl W, et al. A multi-center quality control study of different CA15-3 immunoassays. Clin Lab. 2005;51(11-12):641-5; Haller CA, et al. Comparison of an automated and point-of-care immunoassay to GC-MS for urine oxycodone testing in the clinical laboratory. J Anal Toxicol. 2006 Mar;30(2):106-11; Bayer M, et al. Evaluation of a new enzyme-linked immunosorbent assay for the determination of neopterin. Clin Lab. 2005;51(9-10):495-504; Groche D, et al. Standardization of two immunological HbA1c routine assays according to the new IFCC reference method. Clin Lab. 2003;49(11-12):657-61; Ivan D, et al; German KIMS Board. Applicability of recently established reference values for serum insulin-like growth factor 1: A comparison of two assays--an (automated) chemiluminescence immunoassay and an enzyme-linked immunosorbent assay. Clin Lab. 2005;51(7-8):381-7. Colormetric assays: Kramer KA, et al. Automated spectrophotometric analysis of mitochondrial respiratory chain complex enzyme activities in cultured skin fibroblasts. Clin Chem. 2005 Nov;51(11)):2110-6; Groche D, et al. Standardization of two immunological HbA1c routine assays according to the new IFCC reference method. Clin Lab. 2003; 49(11-I2):657-61; Wolf PL. History of diagnostic enzymology: A review of significant investigations. Clin Chim Acta. 2006 Mar 24.

The TrueMass® analytical platform may also be used for the methods of the invention. TrueMass® is an analytical platform that may be used to get quantitative data from serum or plasma on approximately 400 individual metabolites involved in structural and energetic lipid metabolism such as triglyceride, cholesterol ester and phospholipid metabolism. This platform is useful in profiling diseases as structural and energetic lipids are central components of metabolism and integrated into virtually every biological process in the body. A data set for a plasma or serum sample comprises the quantitative measurement of free cholesterol and the following fatty acids from phosphatidylcholines, phosphatidylethanolamines, lyso-phosphatidylcholines, triglycerides, diglycerides, free fatty acids, and cholesterol esters: 14:0, 15:0, 16:0, 18:0, 20:0, 22:0, 24:0, 14:ln5, 16:ln7, tl6:ln7, 18:ln9, t18:ln9, 18:ln7, 18:2n6, t18:2n6, 18:3n6, 18:3n3, 18:4n3, 20:ln9, 20:2n6, 20:3n9, 20:3n6, 20:4n6, 20:3n3, 20:4n3, 20:5n3, 22:ln9, 22:2n6, 22:4n6, 22:5n3, 22:6n3, 24:ln9, 24:6n3 and plasmalogen derivatives of 16:0, 18:0, 18:ln9 and 18:ln7. Methods for using TrueMass® are known to those of skill in the art, and are also described in the following documents: U.S. Patent Publication No. 2006/0084129 (filed 12/6/05); Mutch DM, et al. An integrative metabolism approach identifies stearoyl-CoA desaturase as a target for an arachidonate-enriched diet. FASEB J. 2005 Apr; 19(6):599-601. Epub 2005 Jan 24; Stone SJ, et al. Lipopenia and skin barrier abnormalities in DGAT2-deficient mice. J Biol Chem. 2004 Mar 19; 279(12):11767-76; Watkins SM, et al. Phosphatidylethanolamine-N-methyltransferase activity and dietary choline regulate liver-plasma lipid flux and essential fatty acid metabolism in mice. J Nutr. 2003 Nov; 133(11):3386-91 ; Watkins SM, et al. Lipid metabolome-wide effects of the PPARgamma agonist rosiglitazone. Lipid Res. 2002 Nov; 43(11):1809-17.

Non-limiting examples of suitable methods may also be found in: U.S. Pat. Publication No. 2004/0143461 and PCT Publication No. WO 03/005628, titled "Generating, Viewing, Interpreting, and Utilizing a Quantitative Database of Metabolites"; Stanton, B. et al. Interaction of estrogen and 2,3,7,8-tetracholorodibenzo-p-dioxin (TCDD) with hepatic fatty acid synthesis and metabolism of male chickens (Gallus domesticus). Comp. Biochem. and Physiology Part C 129 (2001) 137-150; Watkins, S.M. et al. Unique Phospholipid Metabolism in Mouse Heart in Response to Dietary Docosahexaenoic or α-Linoleic Acids. Lipids, Vol. 36, No. 3 (2001) 247-254; and Bernhardt, T.G. et al. Purification of fatty acid ethyl esters by solid-phase extraction and high-performance liquid chromatography. J. of Chromatography B, 675 (1996) 189-196.

As a non-limiting example, the method may include the following steps: extraction, lipid class separation, preparation of fatty acid methyl esters, and fatty acid and sterol separation and quantification. A non-limiting exemplary method includes the following steps: (1) Extractions: The lipids from 200 μL of plasma will be extracted using a modified Folch extraction in chloroform :methanol (2:1 v/v) (Folch, J., M. Lees, et al. (1957). "A simple method for the isolation and purification of total lipides from animal tissues." J Biol Chem 226(1):497-509). Each extraction is performed in the presence of a panel of quantitative authentic internal standards. Extracted lipids are concentrated and prepared for separation by HPLC. (2) Lipid class separation: Individual lipid classes are separated from the extract by HPLC using a variety of methods. Each separated lipid class is collected and dried under nitrogen in preparation for trans-esterification. (3) Preparation of fatty acid methyl esters: Lipid classes are trans-esterified in 3 N methanolic HCl in a sealed vial under a nitrogen atmosphere at 100°C for 45 min. The resulting fatty acid methyl esters are extracted from the mixture with hexane and prepared for automatic injection for gas chromatography by sealing the hexane extracts under nitrogen. (4) Fatty acid and sterol separation and quantification: Fatty acid methyl esters are separated and quantified by capillary gas chromatography using a gas chromatograph (Hewlett-Packard model 6890, Wilmington, DE) equipped with a 30 m DB-225MS capillary column (J&W Scientific, Folsom, CA) and a flame-ionization detector.

Surrogate or internal standards may be used in quantifying the lipid metabolites. Surrogate standards are known in the art. Non-limiting exemplary surrogate standards are described at, inter alia, pages 16-17 and 25-31 of PCT Publication No. WO 03/005628, titled "Generating, Viewing, Interpreting, and Utilizing a Quantitative Database of Metabolites", and in U.S. Patent Publication No. US 2004/0143461. Non-limiting exemplary surrogate standards are also provided below in Table 5.

Table 5. Exemplary Authentic Surrogate Standards

| Metabolite | Abbreviation | Surrogate |
|---|---|---|
| Triglycerides | TGxx | TG17:1n7 |
| Cholesterol Esters | CExx | CE19:0 |
| Free Fatty Acids | FAxx | FA15:1n5 |
| Diglycerides | DGxx | DG17:0 |
| Free Cholesterol | FC | d7-Cholesterol |

(continued)

| Metabolite | Abbreviation | Surrogate |
|---|---|---|
| Phosphatidylcholine | PCxx | PC17:0 |
| Phosphatidylethanolamine | PExx | PE15:1n5 |
| Lysophosphatidylcholine | LYxx | LY17:0 |
| Sphingomyelin | SMxx | SM15:1n5 |
| Prostaglandin $E_2$ | $PGE_2$ | $dPGE_2$ |
| 13,14-dihydro-15-keto Prostaglandin $A_2$ | $PGA_2M$ | $dPGB_2$ |
| Prostaglandin $B_2$ | $PGB_2$ | $dPGB_2$ |
| Prostaglandin $F_{2a}$ | $PGF_{2\alpha}$ | $dPGF_{2\alpha}$ |
| 15-keto-Prostaglandin $F_{2\alpha}$ | 15-keto-$PGF_{2\alpha}$ | $dPGF_{2\alpha}$ |
| 6-keto-Prostaglandin $F_{1\alpha}$ | 6-keto-$PGF_{1\alpha}$ | $dPGF_{2\alpha}$ |
| Thromboxane $B_2$ | $TXB_2$ | $dTXB_2$ |
| 11-dehydro-Thromboxane $B_2$ | $11-DTXB_2$ | $d11-DTXB_2$ |
| Prostaglandin $D_2$ | $PGD_2$ | $dPGD_2$ |
| Prostaglandin $J_2$ | $PGJ_2$ | $dPGB_2$ |
| 15-deoxy-$\Delta$12,14-Prostaglandin $J_2$ | $PGJ_2M$ | $dPGB_2$ |
| 11$\beta$-Prostaglandin $F_{2\alpha}$ | 11$\beta$-$PGF_{2\alpha}$ | $dPGF_{2\alpha}$ |
| 5(S)-Hydroxyeicosatetraenoic acid | 5-HETE | d5-HETE |
| 5(S)-Hydroxyeicosapentaenoic acid | 5-HEPE | d15-HETE |
| Leukotriene $B_4$ | $LTB_4$ | $dLTB_4$ |
| Leukotriene $B_5$ | $LTB_5$ | $dLTB_4$ |
| Leukotriene $C_4$ | $LTC_4$ | $dLTB_4$ |
| Leukotriene $D_4$ | $LTD_4$ | $dLTB_4$ |
| Leukotriene $E_4$ | $LTE_4$ | $dLTB_4$ |
| Leukotriene $F_4$ | $LTF_4$ | $dLTB_4$ |
| 12(S)-Hydroxyeicosatetraenoic acid | 12-HETE | d 12-HETE |
| 12(S)-Hydroxyeicosapentaenoic acid | 12-HEPE | d15-HETE |
| 15(S)-Hydroxyeicosatetraenoicacid | 15-HETE | d15-HETE |
| 15(S)-Hydroxyeicosapentaenoic acid | 15-HEPE | d 15-HETE |
| Lipoxin $A_4$ | $LXA_4$ | $dLTB_4$ |
| 8(S)-Hydroxyeicosatetraenoic acid | 8-HETE | d12-HETE |
| 9-Hydroxyeicosatetraenoic acid | 9-HETE | d12-HETE |
| 11-Hydroxyeicosatetraenoic acid | 11-HETE | d15-HETE |
| 8-iso-Prostaglandin $F_{2\alpha}$ | 8-iso-$PGF_{2\alpha}$ | $dPGF_{2\alpha}$ |
| 9-Hydroxyoctadecadienoic acid | 9-HODE | d9-HODE |
| 13-Hydroxyoctadecadienoic acid | 13-HODE | d13-HODE |
| 20(S)-Hydroxyeicosatetraenoicacid | 20-HETE | d15-HETE |
| 9,10-Epoxyoctadecenoic acid | 9,10-EpOME | d15-HETE |
| 12,13-Epoxyoctadecenoic acid | 12,13-EpOME | d15-HETE |

(continued)

| Metabolite | Abbreviation | Surrogate |
|---|---|---|
| 12,13-Dihydroxyoctadecenoic acid | 12,13-DiHOME | d15-HETE |
| 5,6-Epoxyeicosatrienoic acid | 5,6-EpETrE | d15-HETE |
| 11,12-Epoxyeicosatrienoic acid | 11,12-EpETrE | d1 5-HETE |
| 14,15-Epoxyeicosatrienoic acid | 14,15-EpETrE | d15-HETE |
| 5,6-Dihydroxyeicosatrienoic acid | 5,6-DiHETrE | d 15-HETE |
| 8,9-Dihydroxyeicosatrienoic acid | 8,9-DiHETrE | d15-HETE |
| 11,12-Dihydroxyeicosatrienoic acid | 11,12-DiHETErE | d5-HETE |
| 14,15-Dihydroxyeicosatrienoic acid | 14,15-DiHETrE | d15-HETE |
| 14,15-Epoxyeicosatetraenoic acid | 14,15-EpETE | d15-HETE |
| 17,18-Epoxyeicosatetraenoic acid | 17,18-EpETE | d15HT |
| 14,15-Dihydroxyeicosatetraenoic acid | 14,15-DiHETE | d15HT |
| 17,18-Dihydroxyeicosatetraenoic acid | 1 7,18-DiHETE | d15HT |
| 19,20-Dihydroxydocosapentaenoic acid | 19,20-DiHDPA | d15HT |

Kits

Kits for practicing the methods of the invention are disclosed. The kits include (a) one or more reagents for measuring the amount of one or more lipid metabolites; and (b) instructions for use. A kit may provide 1, 2, 3, 4, 5, 10, 15, 20, or more reagents for measuring the amount of 1, 2, 3, 4, 5, 10, 15, 20, or more lipid metabolites. The kit may further provide one or more reagents for measuring one or more additional biomarkers, such as those disclosed above, and in Tables 2-4. In some embodiments, the kit includes one or more reagents for use in an immunoassay. In some embodiments, the kit includes one or more reagents for use in an MS assay. In some embodiments, the reagent is an antibody. Methods of making antibodies are known to those of ordinary skill in the art.

Disclosed herein is a kit for use in each of the methods described herein, wherein the kit comprises (a) an antibody to a lipid metabolite; and (b) instructions for use. In some embodiments, the kit further comprises: (c) a second antibody to a second lipid metabolite. In some embodiments, the kit further comprises (d) a third antibody to a third lipid metabolite. In some embodiments, the first, second, and/or third lipid metabolite is a fatty acid.

The invention is further illustrated by the following non-limiting examples:

EXAMPLES

MATERIALS AND METHODS FOR EXAMPLES 1-3

Study Populations

The first data set comprised forty-nine (49) liver biopsy samples, which were profiled to determine hepatic triglyceride composition and correlation with hepatic triglyceride concentrations. Among these samples were eight (8) subjects graded as NASH, six (6) subjects graded as NAFLD, and thirty-five (35) normal samples as assessed by a pathological examination of the tissue. These 49 liver samples were collected from males and females of diverse races (white, black, and undefined). Nine of the subjects with normal liver provided matching plasma samples. These samples were used to provide a correlation between liver triglyceride content and plasma lipid metabolites.

A second data set included serum samples from eight subjects with hepatic impairment and eight normal (by liver biopsy) individuals. This data set was used to confirm the findings from the liver biopsy analysis.

Analytical Methods

The lipids from plasma and tissues were extracted in the presence of authentic internal standards by the method of Folch et al. (Folch, J., et al. 1957. A simple method for the isolation and purification of total lipids from animal tissues. J. Biol. Chem. 226:497-509) using chloroform-methanol (2:1, v/v). Plasma 200 μl was used for each analysis. Individual lipid classes within each extract were separated by preparative thin-layer chromatography as described in Watkins, S. M., et al. 2001. Unique phospholipid metabolism in mouse heart in response to dietary docosahexaenoic or {alpha} -linolenic acids. Lipids. 36: 247-254. Authentic lipid class standard compounds were spotted on the two outside lanes of the thin-layer chromatography plate to enable localization of the sample lipid classes. Each lipid fraction was scraped from the plate and trans-esterified in 3 N methanolic-HCl in a sealed vial under a nitrogen atmosphere at 100°C for 45 min. The resulting fatty acid methyl esters were extracted from the mixture with hexane containing 0.05% butylated hydroxytoluene and prepared for gas chromatography by sealing the hexane extracts under nitrogen.

Fatty acid methyl esters were separated and quantified by capillary gas chromatography using a gas chromatograph (Hewlett-Packard model 6890, Wilmington, DE) equipped with a 30 m DB-225MS capillary column (J&W Scientific, Folsom, CA) and a flame-ionization detector as described in Watkins, S. M., et al. 2001. Unique phospholipid metabolism in mouse heart in response to dietary docosahexaenoic or {alpha}-linolenic acids. Lipids. 36: 247-254.

Once a chromatogram was generated, the analytical software (Atlas 2003; Thermo Electron Corporation) identified each analyte lipid metabolite of interest based on the reference standard and generated a raw area. The raw area, peak shape parameters and the response factor for each analyte were exported to an information management system, where an integration algorithm was used to generate the corrected areas for each analyte of interest. Quantitative data were calculated by taking the ratio of the area of the analyte peak to the area of the appropriate surrogate. This ratio was multiplied by the concentration of the surrogate in the original sample to generate data in a microgram per gram of sample format. Each analyte was then divided by its molecular weight and multiplied by 1000 to calculate the nMoles of analyte per gram of sample. Mole percentage data for each lipid class was calculated by dividing the concentration of each fatty acid by the sum of the concentrations of fatty acids within that class.

***Statistical Methods***

Outlier Rejection. Metabolites not detected in more than 30% of subjects were not included in the statistical analysis.

Data. Untransformed mole percentage data were used to correlate with hepatic triglyceride content and was also used for the confirmation of results in the hepatic impairment study.

Correlations. Pearson's correlation coefficient was used to evaluate the relationship of each metabolite with total hepatic triglycerides. The metabolites in Table 6 were correlated with total hepatic triglycerides ($\alpha < 0.2$) and were compared with differences observed in serum between hepatic impaired and normal individuals. Those metabolites that had an opposite effect in hepatic impaired individuals (by an unpaired Student's t-Test on two groups: normal and hepatic impaired) were not included in Table 6.

EXAMPLE I

CORRELATION OF PLASMA AND LIVER FATTY ACID COMPOSITIONS

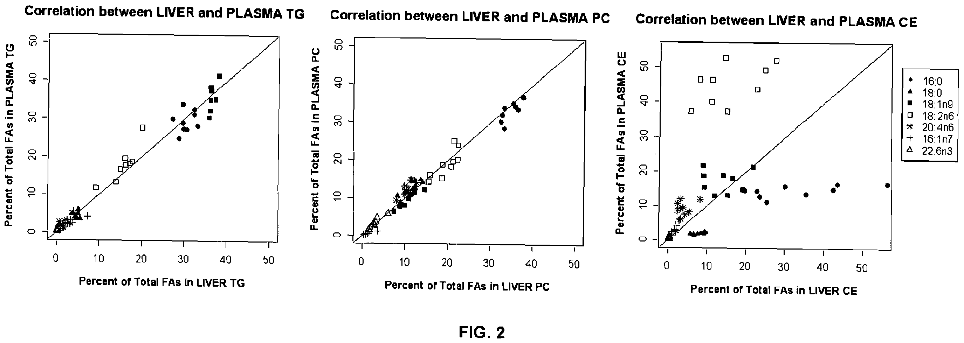

Lipid metabolites expressed as a percentage composition of lipid classes, which correlate to hepatic triglyceride content, were found to be assayable from blood. To determine the potential for blood based measurements to accurately reflect hepatic lipid class fatty acid compositions, we correlated the fatty acid compositions of individual lipid classes from matched plasma-liver samples from the normal humans (from the first set of subjects). The correlation between the composition of blood plasma and liver was excellent for the triglyceride and phosphatidylcholine classes, and in part good for the cholesterol ester class (Fig. 2). This indicated that the blood plasma fatty acid composition of triglyceride and phosphatidylcholine were an accurate indicator of the liver fatty acid composition of triglycerides and phosphatidylcholine, respectively. Thus, blood plasma based measurements of fatty acids may indicate the quantitative amount of triglyceride in the liver (steatosis), provided the compositional data in liver is well-correlated with steatosis.

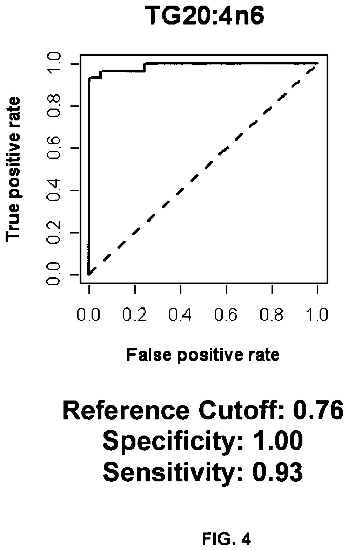

15-20 proportional markers of steatosis were identified in human liver biopsies that provided excellent classification and that were consistent with a single lipid biosynthesis pathway, and predictive of liver triglyceride content. The Receiver Operating Characteristic (ROC) curve for Liver TG20:4n6 is shown in Figure 4.

EXAMPLE 2

CORRELATION BETWEEN HEPATIC STEATOSIS AND HEPATIC FATTY ACID COMPOSITIONS

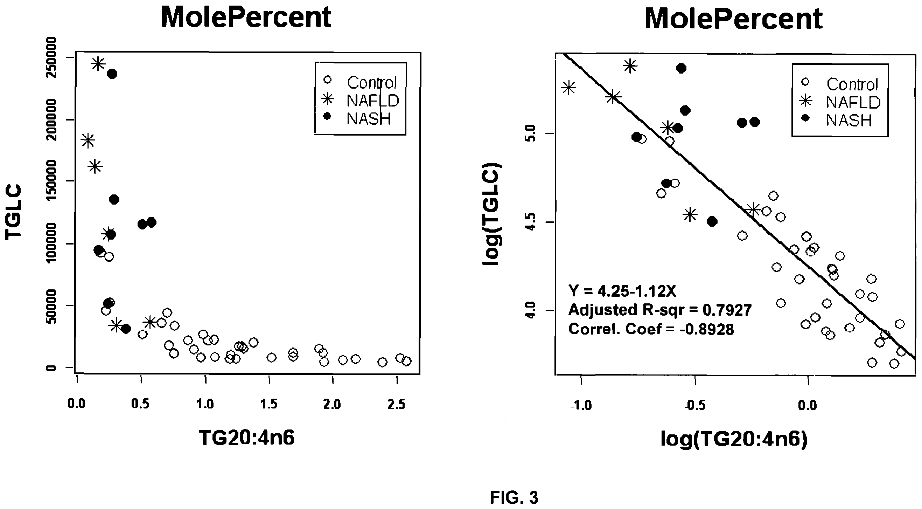

The first data set was used in this experiment. The liver samples of 49 subjects were graded for degree of hepatic steatosis and inflammation. Six subjects were graded as NAFLD and eight subjects were graded as NASH. All other samples were presumed normal. The samples were profiled using TrueMass® technology; many metabolites were found to correlate either positively or negatively with total hepatic triglyceride concentrations. In particular, monounsaturated fatty acids were generally positively correlated with steatosis and essential fatty acids were generally negatively correlated with steatosis. One example of a metabolite that was well-correlated with total hepatic triglycerides was the fatty acid 20:4n6, expressed as a percentage of all fatty acids present in triglycerides (Fig. 3).

Figure 3 shows the relationship between hepatic triglyceride concentrations (nmoles/g) and the relative proportion of 20:4n6 in hepatic triglycerides (expressed as a mole percentage of total triglyceride fatty acids). The relative proportion of TG20:4n6 was an excellent predictor of the total concentration of triglycerides in liver. Despite being graded normal, several normal samples exhibited NAFLD-levels of triglycerides, and the relative proportion of 20:4n6 remained an excellent predictor of total triglyceride concentrations.

EXAMPLE 3

MARKERS OF NAFLD AND NASH

The metabolite markers of NAFLD and NASH in Table 6 were selected based on their observed and/or predicted correlation with the total triglyceride content of liver. Additionally, these markers shown some correlation useful in classifying all 16 subjects tested with normal liver function or hepatic impairment.

Table 6. Blood-based Lipid Metabolite Markers of Hepatic Steatosis (Based on Mole Percentage)

| Lipid Class | Positive Correlates | Negative Correlates |
|---|---|---|
| Triglycerides | TG14:0 | TG15:0 |
| | TG14:1n5 | TG18:2n6 |
| | TG16:0 | TG18:3n3 |
| | TG18:1n7 | TG20:0 |
| | TGMUFA | TG20:2n6 |
| | TGn7 | TG20:3n6 |
| | TGSFA | TG20:3n9 |
| | TG16:1n7 | TG20:4n6 |
| | | TG20:5n3 |
| | | TG22:0 |
| | | TG22:1n9 |
| | | TG22:2n6 |
| | | TG22:4n6 |
| | | TG22:5n3 |
| | | TG22:5n6 |
| | | TG22:6n3 |
| | | TG24:0 |
| | | TG24:1n9 |
| | | TGn3 |
| | | TGn6 |
| | | TGPUFA |
| Free Fatty Acids | | FA16:1n7 |
| Phospho-tidylcholines | PC14:0 | PC18:1n7 |
| | PC16:1n7 | PC20:4n6 |
| | PC18:1n7 | PC22:5n6 |
| | PC18:1n9 | PCn6 |

(continued)

| Lipid Class | Positive Correlates | Negative Correlates |
|---|---|---|
| | PC18:3n3 | PCPUFA |
| | PC18:3n6 | PC22:5n3 |
| | PC 18:4n3 | |
| | PC20:0 | |
| | PC20:1n9 | |
| | PC20:2n6 | |
| | PC20:3n6 | |
| | PC20:4n3 | |
| | PC20:5n3 | |
| | PC22:0 | |
| | PC22:1n9 | |
| | PC24:0 | |
| | PC24:1 n9 | |
| | PCdm | |
| | PCdm 18:0 | |
| | PCdm18:1n7 | |
| | PCSFA | |
| Phospho-tidylethanol-amines | | PE20:4n6 |
| Cholesterol Esters | CE16:1n7 | CE14:1n5 |
| | CE18:1n7 | CE18:0 |
| | CE18:1n9 | CE20:0 |
| | CE18:2n6 | CE20:1 n9 |
| | CE18:3n6 | CE20:2n6 |
| | CE22:5n3 | CE20:3n9 |
| | CE22:6n3 | CE20:4n3 |
| | CEMUFA | CE20:4n6 |
| | CEn6 | CE22:0 |
| | CEn7 | CE22:2n6 |
| | CEPUFA | CE24:0 |
| | CE14:0 | CESFA |
| Total Fatty Acids | 14:0 | 15:0 |
| | 16:0 | 20:0 |
| | 18:0 | 22:0 |
| | 16:1n7 | 18:2n6 |
| | 18:1n7 | 20:2n6 |
| | 18:1n9 | 20:3n9 |
| | 18:3n6 | 20:4n3 |
| | 18:4n3 | 20:4n6 |

(continued)

| Lipid Class | Positive Correlates | Negative Correlates |
|---|---|---|
| | 18:4n3 | 22:4n6 |
| | | 22:5n6 |

EXAMPLE 4

FATTY ACID MARKERS OF NAFLD AND NASH IN PLASMA

**Study Population**

A set of NASH, NAFLD, and normal control plasma samples were collected to examine the differences in the lipid composition in plasma. There were 30 NASH patients, 7 NAFLD patients, and 12 normal controls.

**Analytical Methods**

Lipid metabolites were quantified from fasted plasma, serum and liver samples. Lipids measured included cholesterol, cholesterol esters (CE), diglycerides (DG), free cholesterol (FS), free fatty acids (FA), lysophosphatidylcholine (LY), phosphatidylcholine (PC), phosphatidylethanolamine (PE) and triglycerides (TG). For CE, DG, FA, LY, PC, PE and TG lipid classes the following fatty acid components were quantified as a proportion of total fatty acids within the lipid class: 14:0, 15:0, 16:0, 18:0, 20:0, 22:0, 24:0, 14:1n5, 16:1n7, 18:1n7, 20:1n9, 20:3n9, 22:1n9, 24:1n9, 18:2n6, 18:3n6, 20:2n6, 20:3n6, 20:4n6, 22:2n6, 22:4n6, 22:5n6, 18:3n3, 18:4n3, 20:3n3, 20:4n3, 20:5n3, 22:5n3, 22:6n3, 24:6n3, plasmalogen derivatives of 16:0, 18:0, 18:1n7 and t16:1n7 t18:1n9t18:2n6. In this example, the term "LC" indicates the value shown is the total concentration of the lipid class expressed as nMoles per gram of serum or plasma. Thus, in this example, the abbreviation PC18:2n6 indicates the percentage of plasma or serum phosphatidylcholine comprised of linoleic acid (18:2n6), the term TGLC indicates the absolute amount (in nMoles per gram) of triglyceride present in plasma or serum.

The lipids from the sample were extracted in the presence of authentic surrogate standards for each lipid class by a liquid:liquid extraction, creating a lipid extract. The mass of the sample and surrogate were recorded at this step in order to accurately determine the amount of material being analyzed. The mass of the sample and the surrogate standards were used to calculate the quantitative amount of each fatty acid in each lipid class.

The neutral and phospholipid classes were separated from one another via a solid phase extraction with a Varian Vac Elut 20 vacuum manifold and Supelco LC-SI silica packed SPE cartridges. Once these extracts were prepared, the neutral lipid classes were separated by preparative thin layer chromatography on silica gel G-60 TLC plates. The phospholipid classes were separated via high performance liquid chromatography on an Agilent 1100 Series HPLC, with a Phenomenex Sperex 5u OH Diol column (250 x 4.6mm, 5 micron) and a SEDEX 75 evaporative light scattering detector. Once each class was isolated, the lipid class was trans-esterified with 1% sulfuric acid in methanol, resulting in the formation of fatty acid methyl esters (FAMEs). The FAME mixture for each class was separated and quantified by capillary gas chromatography (GC) on an Agilent GC6890, with a J&W Scientific HP-88 fused silica capillary column (30m x 25um, 0.2 um film) and a flame-ionization detector.

**Statistical Methods**

Mole percentage data and lipid class concentrations were evaluated for markers of NAFLD and NASH. Data were not transformed for the analysis. Metabolites not detected in more than 30% of subjects were not included in the statistical analysis. Two-tailed t-tests were used to compare the groups (NASH vs. Normals, and NAFLD vs. Normals).

**Results**

Tables 7 and 8 show markers significantly associated with NASH and NAFLD, respectively. Most lipid classes in NASH and NAFLD subjects did not differ significantly from normal. Phosphatidylethanolamine and phosphatidylcholine were significantly decreased in NASH relative to normal (p-values from t-test: 0.001, 0.021). Phosphatidylcholine and lysophosphatidylcholine were significantly decreased in NAFLD relative to normal (p-values from t-test: 0.05, 0.042). Very similar results were obtained from the non-parametric Wilcoxon test.

Omega 3 fatty acids were decreased, particularly DHA, in NASH subjects relative normal controls. Decreases in DHA were seen in NASH relative to normal controls both quantitatively and compositionally in CE, PC and PE. DHA was significantly decreased in NASH in FA, LY, and TG only compositionally. 18:3n3 was significantly decreased in PC both quantitatively and compositionally, while it was only significantly reduced compositionally in free fatty acids. 22:5n3 was significantly increased compositionally in PC in NASH and NAFLD relative to normal subjects.

While 18:2n6 was quantitatively significantly decreased in phospholipids in NASH and NAFLD relative to normal subjects, 20:3n6 was significantly increased in CE, FA, and TG. Compositionally, 18:2n6 was significantly decreased only in LY, while 20:3n6 was increased in every lipid class except DG in NASH and NAFLD relative normal subjects.

Saturated fatty acids were significantly increased in NASH relative to normal controls in PE, PC and DG. NAFLD subjects also tended to have higher saturated fatty acids than normal controls. Compositionally, only 18:0 was increased in NASH and NAFLD in CE, LY, and PC.

Table 7. NASH markers (significant in t test at .1. p value is shown in parentheses)

| Lipid Class | Increased from Normal | Decreased from Normal |
|---|---|---|
| **Diacylglycerol** | DG20:3n6 (0.0868)<br>DG22:5n6 (0.0418) | |
| **Triacylglycerol** | TG18:1n7 (0.0709)<br>TG20:3n6 (0.0025) | TG18:3n3 (0.0934)<br>TG20:3n9 (0.0999)<br>TG22:6n3 (0.0364)<br>TG24:0 (0.0602) |
| **Free fatty acid** | FA18:1n7 (0.0015)<br>FA18:1n9 (0.0018)<br>FA20:3n6 (0.0123) | FA16:0 (0.0448)<br>FA18:3n3 (0.0051)<br>FA22:6n3 (0.0018) |
| **Phospholipids** | PC18:0 (0.004)<br>PC18:3n6 (0.0181)<br>PC20:3n6 (0.0001)<br>PC20:4n3 (0.0219)<br>PC22:1n9 (0.0503)<br>PC22:4n6 (0.0002)<br>PC22:5n3 (0.016)<br>PC22:5n6 (0.0147)<br>PCdm18:1n7 (0.0805)<br>PE18:3n6 (0.0409)<br>PE20:1n9 (0.0432)<br>PE20:3n6 (0.0044)<br>PE22:4n6 (0.0016)<br>PE22:5n3 (0.0694) | PCLC (0.001)<br>PC18:3n3 (0.0378)<br>PC22:6n3 (0.0405)<br>PELC (0.0211)<br>PE14:0 (0.0674)<br>PE22:6n3 (0.0025) |
| **Cholesterol Esters** | CE18:0 (0.0266)<br>CE18:1n7 (0.0986)<br>CE18:3n6 (0.0752)<br>CE20:3n6 (0.0001)<br>CE24:0 (0.0855) | CE22:6n3 (0.0345) |
| **Sphingomyelin** | SP16:1n7 (0.0695)<br>SP18:0 (0.0994)<br>SP20:3n6 (0.0112)<br>SP22:1n9 (0.0038) | SP22:6n3 (0.0651) |
| **Lysophosphatidylcholine** | LY16:0 (0.0557)<br>LY18:0 (0.0031)<br>LY20:3n6 (0.0076)<br>LY20:3n9 (0.0737)<br>LY20:4n3 (0.0441) | LY18:1n7 (0.086)<br>LY18:1n9 (0.036)<br>LY18:2n6 (0.0004)<br>LY18:3n3 (0.0703)<br>LY22:6n3 (0.0323) |

Table 8. NAFLD markers (significant in t test at.1. p value is shown in parentheses)

| Lipid Class | Increased from Normal | Decreased from Normal |
|---|---|---|
| Diacylglycerol | DG20:3n6 (0.0516) | DG22:1n9 (0.0304) |
| Triacylglycerol | TG20:3n6 (0.0226)<br>TG22:5n3 (0.0814) | TG22:2n6 (0.0702) |
| Free fatty acid | FA18:1n9 (0.0192)<br>FA20:3n6 (0.0244)<br>FA22:5n3 (0.0166) | |
| Phospholipids | PC18:0 (0.006)<br>PC18:3n6 (0.0156)<br>PC20:3n6 (0.0003)<br>PC20:4n3 (0.0214)<br>PC22:5n3 (0.0037) | PCLC (0.0315)<br>PC 18:1n7 (0.0058)<br>PC18:2n6 (0.0714)<br>PC20:2n6 (0.0035) |
| Cholesterol Esters | CE18:3n6 (0.0139)<br>CE20:3n6 (0.0018) | |
| Sphingomyelin | SP20:3n6 (0.0164) | SP16:0 (0.037) |
| Lysophosphatidylcholine | LY15:0(0.0659)<br>LY18:0 (0.0959)<br>LY20:3n6 (0.0004) | LYLC (0.0419)<br>LY18:1n7 (0.0017)<br>LY18:2n6 (0.0025) |

Contrary to what was identified in Example 3, in this particular study, the following metabolites were not found to be positively associated with steatosis: PC 20:2n6; PC18:1n7; and CE22:6n3. Furthermore, in this study, contrary to what was identified in Example 3, the following metabolites were not found to be negatively associated with steatosis: TG20:3n6; TG22:5n3; and PC22:5n3.

EXAMPLE 5

EICOSANOID MARKERS OF NAFLD AND NASH IN PLASMA

**Study Population**

A subset of the study population used in Study Three was used to determine differences in the lipid composition of Eicosanoids between NASH, NAFLD and normal subjects. There were 26 NASH patients, 5 NAFLD patients, and 12 normal controls.

**Analytical Methods**

The eicosanoids from 250 $\mu$L of plasma or serum were extracted using protein precipitation and filtering prior to loading on an LC/MS. Twenty microliters of a mixture of deuterated surrogates for quantitation was added to each sample and thoroughly mixed. To each plasma/serum sample 10 $\mu$L antioxidant solution (0.2mg/mL BHT.EDTA in 50:50 MeOH:H2O) was added and thoroughly mixed. Protein precipitation was carried out by adding 1 mL methanol to each sample followed by mixing. The samples were centrifuged at - 4°C and 17000g for 10 minutes. The supernatants were dried under nitrogen for 2 hours at 10psi. Dried samples were reconstituted with 60ul methanol:deionized water (50:50). After mixing, samples were transferred to silanized autosampler inserts for LC/MSMS analysis. The samples were injected onto an Agilent Stable Bond C18 column (150x2.1mm, 1.8 micron) connected to an Applied Biosystems 4000 QTRAP. The analytes were ionized via negative electrospray and the mass spectrometer was operated in the tandem MS mode.

Abbreviations for a number of eicosanoids are provided in Table 9 below.

Table 9. Eicosanoids

| Metabolite | Abbreviation |
|---|---|
| Prostaglandin $E_2$ | PGE$_2$ or PGE2 |
| 13,14-dihydro-15-keto Prostaglandin $A_2$ | PGA$_2$M or PGA2M |
| Prostaglandin $B_2$ | PGB$_2$ or PGB2 |
| Prostaglandin $F_{2a}$ | PGF$_{2\alpha}$ or PGF2$\alpha$ |
| 15-keto-Prostaglandin $F_{2\alpha}$ | 15-keto-PGF$_{2\alpha}$ or 15-keto-PGF2$\alpha$ |
| 6-keto-Prostaglandin $F_{1\alpha}$ | 6-keto-PGF$_{1\alpha}$ or 6-keto-PGF1$\alpha$ |
| Thromboxane $B_2$ | TXB$_2$ or TXB2 |
| 11-dehydro-Thromboxane $B_2$ | 11-DTXB$_2$ or 11-DTXB2 |
| Prostaglandin $D_2$ | PGD$_2$ or PGD2 |
| Prostaglandin $J_2$ | PGJ$_2$ or PGJ2 |
| 15-deoxy-$\Delta$12,14-Prestaglandin $J_2$ | PGJ$_2$M or PGJ2M |
| 11$\beta$-Prostaglandin $F_{2\alpha}$ | 11$\beta$-PGF$_{2\alpha}$ or 11$\beta$-PGF2$\alpha$ |
| 5(S)-Hydroxyeicosatetraenoic acid | 5-HETE |
| 5(S)-Hydroxyeicosapentaenoic acid | 5-HEPE |
| Leukotriene $B_4$ | LTB$_4$ or LTB4 |
| Leukotriene $B_5$ | LTB$_5$ or LTB5 |
| Leukotriene $C_4$ | LTC$_4$ or LTC4 |
| Leukotriene $D_4$ | LTD$_4$ or LTD4 |
| Leukotriene $E_4$ | LTE$_4$ or LTE4 |
| Leukotriene $F_4$ | LTF$_4$ or LTF4 |
| 12(S)-Hydroxyeicosatetraenoic acid | 12-HETE |
| 12(S)-Hydroxyeicosapentaenoic acid | 12-HEPE |
| 15(S)-Hydroxyeicosatetraenoic acid | 15-HETE |
| 15(S)-Hydroxyeicosapentaenoic acid | 15-HEPE |
| Lipoxin $A_4$ | LXA$_4$ or LXA4 |
| 8(S)-Hydroxyeicosatetraenoic acid | 8-HETE |
| 9-Hydroxyeicosatetraenoic acid | 9-HETE |
| 11-Hydroxyeicosatetraenoic acid | 11-HETE |
| 8-iso-Prostaglandin $F_{2\alpha}$ | 8-iso-PGF$_{2a}$ or 8-iso-PGF2a |
| 9-Hydroxyoctadecadienoic acid | 9-HODE |
| 13-Hydroxyoctadecadienoic acid | 13-HODE |
| 20(S)-Hydroxyeicosatetraenoic acid | 20-HETE |
| 9,10-Epoxyoctadecenoic acid | 9,10-EpOME |
| 12,13-Epoxyoctadecenoic acid | 12,13-EpOME |
| 12,13-Dihydroxyoctadecenoic acid | 12,13-DiHOME |
| 5,6-Epoxyeicosatrienoic acid | 5,6-EpETrE |
| 11,12-Epoxyeicosatrienoic acid | 11,12-EpETrE |
| 14,15-Epoxyeicosatrienoic acid | 14,15-EpETrE |

(continued)

| Metabolite | Abbreviation |
|---|---|
| 5,6-Dihydroxyeicosatrienoic acid | 5,6-DiHETrE |
| 8,9-Dihydroxyeicosatrienoic acid | 8,9-DiHETrE |
| 11,12-Dihydroxyeicosatrienoic acid | 11,12-DiHETErE |
| 14,15-Dihydroxyeicosatrienoic acid | 14,15-DiHETrE |
| 14,15-Epoxyeicosatetraenoic acid | 14,15-EpETE |
| 17,18-Epoxyeicosatetraenoic acid | 17,18-EpETE |
| 14,15-Dihydroxyeicosatetraenoic acid | 14,15-DiHETE |
| 17,18-Dihydroxyeicosatetraenoic acid | 17,18-DiHETE |
| 19,20-Dihydroxydocosapentaenoic acid | 19,20-DiHDPA |

**Statistical Methods**

Quantitative data (pMoles per gram of plasma) were evaluated for markers of NAFLD and NASH. Quantitative data were not transformed for the analysis. Metabolites not detected in more than 30% of subjects were not included in the statistical analysis. Two-tailed t-tests were used to compare the groups (NASH vs. Normals, and NAFLD vs. Normals).

**Results**

Table 10 shows the eicosanoid metabolites that were significantly associated with NASH and NAFLD. The NAFLD group have significantly higher 13,14-dihydro-15-keto Prostaglandin $A_2$, 11-dehydro-Thromboxane $B_2$, and 12,13-Dihydroxyoctadecenoic acid. 19,20-Dihydoxydocosapentaenoic acid was significantly decreased according to the t-test but did not reach significance by the Wilcoxon test.

The NASH group had significantly higher Prostaglandin $E_2$, 15-keto-Prostaglandin $F_{2\square}$, and Leukotriene $D_4$ as assessed by the Wilcoxon test, but did not reach significance by t-test. HETE's, including 5-HETE, 8-HETE, 9-HETE, 11-HETE, 12-HETE, and 15-HETE, were significantly increased in NASH over normal in both tests. 11-HETE and 15-HETE were linearly anti-correlated, but more strongly anti-correlated on a log scale, with DHA in a few lipid classes.

Table 10. NASH and NAFLD markers (significant in t test at .1. p value is shown in parentheses)

| NASH | | NAFLD | |
|---|---|---|---|
| Increased | Decreased | Increased | Decreased |
| $PGB_2$ (0.08 15) | 19,20-DiHDPA (0.063) | 15-HETE (0.0937) | $PGA_2M$ (0.0296) |
| $PGE_2$ (0.0627) | | | 6-keto-$PGF_{1\alpha}$ (0.0896) |
| $PGF_{2\alpha}$ (0.0542) | | | 11-DTXB$_2$ (0.0027) |
| 15-keto-$PGF_{2\alpha}$ (0.0603) | | | 12,13-DiHOME (0.009) |
| 5-HETE (0.019) | | | 9, 10-EpOME (0.0785) |
| 8-HETE (0.0012) | | | 12,13-EpOME (0.0977) |
| 9-HETE (0.0031) | | | 19,20-DiHDPA (0.0297) |
| 11-HETE (0.0001) | | | 8-iso-$PGF_{2\alpha}$ (0.0976) |
| 12-HETE (0.0206) | | | |
| 15-HETE (0.0001) | | | |
| 12-HEPE (0.0902) | | | |
| 11,12-EpETrE (0.0744) | | | |
| 8,9-DiHETrE (0.0004) | | | |

EXAMPLE 6

CLASSIFICATIONS BASED ON MARKER COMBINATIONS

Diagnostics for NASH can be built either from the described marker metabolites directly or from simple combinations of these markers. As each diagnostic application requires unique performance characteristics, metabolite concentrations can be combined into simple algorithms to provide the sensitivity and specificity required for a particular desired test.

Results from Example 4 and Example 5 were used to develop classifiers for distinguishing NASH from NAFLD and Normal subjects. Linear combinations of metabolite pairs were evaluated for their ability to classify NASH versus NAFLD using a receiver operator curve (ROC). Performance characteristics evaluated in this experiment included the area under the ROC curve (ROC AUC), the sensitivity and the specificity of the test. Examples of combinations that provided overall sensitivity and specificity (Combination 1), high sensitivity with less specificity (Combinations 2 and 3), and high specificity with less sensitivity (Combinations 4 and 5) are shown in Table 11 below.

Although a linear combination of metabolites was chosen as the algorithmic method for this example, any algorithm (including ratios, etc.) can be used to generate a test variable from the claimed metabolites.

Table 11. Performance of linear combinations of metabolites in classifying NASH from NAFLD and Normal subjects

|  | Metabolite Pair | ROC AUC | Sensitivity | Specificity | Threshold |
|---|---|---|---|---|---|
| 1. | 15-HETE \| 15-keto-PGF$_{2\alpha}$ | 0.90 | 0.88 | 0.94 | 0.58 |
| 2. | TG18:1n7 \| PC20:3n6 | 0.81 | 0.97 | 0.58 | 0.34 |
| 3. | 11-HETE \| CE22.6n3 | 0.82 | 1.00 | 0.56 | 0.34 |
| 4. | 11-HETE \| PCTL | 0.87 | 0.60 | 1.00 | 0.81 |
| 5. | PC22:6n3 \| PC18:3n3 | 0.83 | 0.60 | 1.00 | 0.74 |

The desired test performance will depend on the application (for instance if a subject is to undergo an invasive procedure on the basis of the test, it may be most useful to ensure a high-degree of specificity). The performance of the test can be modulated by choosing the individual metabolites components of the algorithm and the threshold (critical value) for classification. The metabolites chosen for inclusion in the algorithm may be any of the eicosanoids or fatty acid markers described herein or any of the following acylcarnitines, sterols, bile acids or oxysterols: **Carnitine Metabolites and Acylcarnitines:** L-Carnitine, g-Butyrobetaine; Trimethyllysine; Acetylcarnitine; Propionylcarnitine; Butyrylcarnitine; Valerylcarnitine; Hexanoylcarnitine; Octanoylcarnitine; Decanoylcarnitine; Dodecanoylcarnitine; Myristoylcarnitine; Palmitoylcarnitine; Stearoylcarnitine; Oleoylcamitine; Linoleoylcarnitine. **Sterols, Bile Acids and Oxysterols:** Cholesterol; 7-Dehydrocholesterol; Desmosterol; Lanosterol; Lathasterol; Cholestanol; Coprostanol; b-Sitosterol; Campesterol; Stigmasterol; 4-Cholesten-7a-ol-3-one; 7a-Hydroxycholesterol; 27-Hydroxycholesterol; 25-Hydroxycholesterol; 24S-Hydroxycholesterol; 4b-hydroxycholesterol; Cholic acid; Chenodeoxycholic acid; Deoxycholic acid; Lithocholic acid; Glycocholic acid; Glycochenodeoxycholic acid; Glycodeoxycholic acid; Glycolithocholic acid; Taurocholic acid; Taurochenodeoxycholic acid; Taurodeoxycholic acid; Taurolithocholic acid; Ursodeoxycholic acid; Glycoursodeoxycholic acid.

**Claims**

1.   A method of diagnosing or monitoring non-alcoholic steatohepatitis (NASH) in a subject, comprising:

(A) determining an amount of one or more lipid metabolites in one or more blood, serum, or plasma samples from the subject, wherein:

the lipid metabolites PC22:6n3, PCLC, PC18:3n3, TG18:3n3, TG20:3n9, TG22:6n3, TG24:0, PE22:6n3, LY22:6n3, PE14:0, PELC, FA16:0, CE22:6n3, LY18:1n9, LY18:2n6, LY18:3n3, and/or 19,20-DiHDPA are negatively associated with NASH; and
the lipid metabolites PC22:5n6, PC18:3n6, PC20:3n6, PC20:4n3, PC22:1n9, PCdml8:ln7, PC18:0, PC22:5n3, CE 18:0, CE24:0, CE18:1n7, CE18:3n6, TG18:1n7, LY16:0, FA18:1n7, SM18:0, SM22:1n9, CE20:3n6, TG20:3n6, LY18:0, LY20:3n6, PE18:3n6, PE20:3n6, PE22:5n3, FA18:1n9, FA20:3n6, 15-HETE, PGB2, PGE2, PGF2α, 15-keto-PGF2α, 5-HETE, 8-HETE, 9-HETE, 11-HETE, 12-HETE, 12-HEPE, 11,12-EpETrE, and/or 8,9-DiHETrE, are positively associated with NASH; and

(B) correlating the amount(s) of the one or more lipid metabolites with the presence, absence or progression of non-alcoholic steatohepatitis (NASH).

2. The method of claim 1 wherein the one or more lipid metabolites comprise one or more fatty acids.

3. The method of claim 2, wherein the amount(s) of the one or more fatty acids are relative amount(s) of the one or more fatty acids to total fatty acid content in the lipids of one or more lipid classes in one or more samples.

4. The method of claim 2, wherein the method comprises:: determining a relative amount of one or more fatty acids to total fatty acid content in the lipid metabolites of one or more lipid classes in the blood, serum, or plasma samples from the subject; and correlating the relative amount(s) with the presence of - non-alcoholic steatohepatitis (NASH).

5. The method of any one of the preceding claims, further comprising comparing the amount(s) of the one or more lipid metabolites to one or more references.

6. The method of any one of the preceding claims, wherein the amounts of two or more of the lipid metabolites are determined.

7. The method of any one of the preceding claims, wherein the method of monitoring is used to determine the subject's response to treatment.

8. The method of any one of claims 1-7, wherein the method of monitoring comprises assessing the progression of non-alcoholic steatohepatitis (NASH).

9. The method of any one of the preceding claims, wherein the subject is a liver graft donor candidate, is being evaluated for bariatric surgery, a liver transplant, or has had bariatric surgery.

10. The method of any one of the preceding claims, wherein mathematical formulas or models are used to quantify the effect of each lipid metabolite of the one or more lipid metabolites.

11. The method of any one of the preceding claims, wherein the subject is a human.

12. The method of any of the preceding claims, wherein the method of monitoring is used to determine the subject's response to treatment.

**Patentansprüche**

1. Verfahren zur Diagnose oder Kontrolle nichtalkoholischer Steatohepatitis (NASH) bei einem Subjekt, bei dem man:

(A) die Menge eines oder mehrerer Lipidmetaboliten in einer oder mehreren Blut-, Serum- oder Plasmaproben von dem Subjekt bestimmt, wobei:

die Lipidmetaboliten PC22:6n3, PCLC, PC18:3n3, TG18:3n3, TG20:3n9, TG22:6n3, TG24:0, PE22:6n3, LY22:6n3, PE14:0, PELC, FA16:0, CE22:6n3, LY18:1n9, LY18:2n6, LY18:3n3 und/oder 19,20DiHDPA negativ mit NASH assoziiert sind und
die Lipidmetaboliten PC22:5n6, PC18:3n6, PC20:3n6, PC20:4n3, PC22:1n9, PCdm18:1n7, PC18:0, PC22:5n3, CE 18:0, CE24:0, CE18:1n7, CE18:3n6, TG18:1n7, LY16:0, FA18:1n7, SM18:0, SM22:1n9, CE20:3n6, TG20:3n6, LY18:0, LY20:3n6, PE18:3n6, PE20:3n6, PE22:5n3, FA18:1n9, FA20:3n6, 15-HETE, PGB2, PGE2, PGF2α, 15-keto-PGF2α, 5-HETE, 8-HETE, 9-HETE, 11-HETE, 12-HETE, 12-HEPE, 11,12-EpETrE und/oder 8,9-DiHETrE positiv mit NASH assoziiert sind, und

(B) die Menge(n) des einen oder der mehreren Lipidmetaboliten mit dem Vorliegen, der Abwesenheit oder dem Fortschreiten von nichtalkoholischer Steatohepatitis (NASH) korreliert.

**2.** Verfahren nach Anspruch 1, wobei der eine oder die mehreren Lipidmetabolit(en) eine oder mehrere Fettsäuren umfasst/umfassen.

**3.** Verfahren nach Anspruch 2, wobei die Menge(n) der einen oder mehreren Fettsäuren die relative(n) Menge(n) der einen oder mehreren Fettsäuren, bezogen auf den Gesamtfettsäuregehalt, in den Lipiden von einer oder mehreren Lipidklassen in einer oder mehreren Proben ist/sind.

**4.** Verfahren nach Anspruch 2, wobei das Verfahren Folgendes umfasst: das Bestimmen einer relativen Menge einer oder mehrerer Fettsäuren, bezogen auf den Gesamtfettsäuregehalt, in den Lipidmetaboliten der einen oder mehreren Lipidklassen in den Blut-, Serum- oder Plasmaproben von dem Subjekt und das Korrelieren der relativen Menge(n) mit dem Vorliegen von nichtalkoholischer Steatohepatitis (NASH).

**5.** Verfahren nach einem der vorhergehenden Ansprüche, bei dem man weiterhin die Menge (n) des einen oder der mehreren Lipidmetaboliten mit einem oder mehreren Vergleichswerten vergleicht.

**6.** Verfahren nach einem der vorhergehenden Ansprüche, bei dem man die Mengen von zwei oder mehr der Lipidmetaboliten bestimmt.

**7.** Verfahren nach einem der vorhergehenden Ansprüche, wobei man das Kontrollverfahren anwendet, um die Reaktion des Subjekts auf eine Behandlung festzustellen.

**8.** Verfahren nach einem der Ansprüche 1-7, wobei man bei dem Kontrollverfahren das Fortschreiten von nichtalkoholischer Steatohepatitis (NASH) untersucht.

**9.** Verfahren nach einem der vorhergehenden Ansprüche, wobei es sich bei dem Subjekt um einen Kandidaten für die Spende eines Lebertransplantats handelt, das Subjekt auf eine Eignung für einen bariatrischen Eingriff oder eine Lebertransplantation untersucht wird oder das Subjekt sich einem bariatrischen Eingriff unterzogen hat.

**10.** Verfahren nach einem der vorhergehenden Ansprüche, bei dem man zur Quantifizierung der Wirkung der einzelnen Lipidmetaboliten von dem einen oder den mehreren Lipidmetaboliten mathematische Formeln oder Modelle anwendet.

**11.** Verfahren nach einem der vorhergehenden Ansprüche, wobei das Subjekt ein Mensch ist.

**12.** Verfahren nach einem der vorhergehenden Ansprüche, wobei man das Kontrollverfahren anwendet, um die Reaktion des Subjekts auf eine Behandlung festzustellen.

**Revendications**

**1.** Méthode de diagnostic ou de suivi de la stéatohépatite non alcoolique (NASH) chez un sujet, comprenant :

(A) la détermination d'une quantité d'un ou de plusieurs métabolites lipidiques dans un ou plusieurs échantillons de sang, de sérum ou de plasma issus du sujet, dans laquelle :

les métabolites lipidiques PC22:6n3, PCLC, PC18:3n3, TG18:3n3, TG20:3n9, TG22:6n3, TG24:0, PE22:6n3, LY22:6n3, PE14:0, PELC, FA16:0, CE22:6n3, LY18:1n9, LY18:2n6, LY18:3n3, et/ou 19,20-DiHDPA sont associés négativement à la NASH ; et
les métabolites lipidiques PC22:5n6, PC18:3n6, PC20:3n6, PC20:4n3, PC22:1n9, PCdm18:1n7, PC18:0, PC22:5n3, CE 18:0, CE24:0, CE18:1n7, CE18:3n6, TG18:1n7, LY16:0, FA18:1n7, SM18:0, SM22:1n9, CE20:3n6, TG20:3n6, LY18:0, LY20:3n6, PE18:3n6, PE20:3n6, PE22:5n3, FA18:1n9, FA20:3n6, 15-HETE, PGB2, PGE2, PGF2α, 15-kéto-PGF2α, 5-HETE, 8-HETE, 9-HETE, 11-HETE, 12-HETE, 12-HEPE, 11,12-EpETrE, et/ou 8,9-DiHETrE, sont associés positivement à la NASH ; et

(B) la corrélation de la ou des quantités du ou des métabolites lipidiques à la présence, l'absence ou la progression de la stéatohépatite non alcoolique (NASH).

**2.** Méthode selon la revendication 1 dans laquelle le ou les métabolites lipidiques comprennent un ou plusieurs acides gras.

3. Méthode selon la revendication 2, dans laquelle la ou les quantités du ou des acides gras sont une ou plusieurs quantités relatives du ou des acides gras par rapport à la teneur totale en acides gras dans les lipides d'une ou de plusieurs classes lipidiques dans un ou plusieurs échantillons.

4. Méthode selon la revendication 2, dans laquelle la méthode comprend : la détermination d'une quantité relative d'un ou de plusieurs acides gras par rapport à la teneur totale en acides gras dans les métabolites lipidiques d'une ou de plusieurs classes lipidiques dans les échantillons de sang, de sérum ou de plasma issus du sujet ; et la corrélation de la ou des quantités relatives à la présence de la stéatohépatite non alcoolique (NASH).

5. Méthode selon l'une quelconque des revendications précédentes, comprenant en outre la comparaison de la ou des quantités du ou des métabolites lipidiques à une ou plusieurs références.

6. Méthode selon l'une quelconque des revendications précédentes, dans laquelle les quantités de deux des métabolites lipidiques ou plus sont déterminées.

7. Méthode selon l'une quelconque des revendications précédentes, dans laquelle la méthode de suivi est utilisée pour déterminer la réponse du sujet au traitement.

8. Méthode selon l'une quelconque des revendications 1 à 7, dans laquelle la méthode de suivi comprend l'évaluation de la progression de la stéatohépatite non alcoolique (NASH).

9. Méthode selon l'une quelconque des revendications précédentes, dans lequel le sujet est un candidat donneur de greffon de foie, est sujet à une évaluation pour chirurgie bariatrique, une greffe de foie, ou a subi une chirurgie bariatrique.

10. Méthode selon l'une quelconque des revendications précédentes, dans laquelle des modèles ou formules mathématiques sont utilisés pour quantifier l'effet de chaque métabolite lipidique du ou des métabolites lipidiques.

11. Méthode selon l'une quelconque des revendications précédentes, dans laquelle le sujet est un humain.

12. Méthode selon l'une quelconque des revendications précédentes, dans laquelle la méthode de suivi est utilisée pour déterminer la réponse du sujet au traitement.

**FIG. 1**

FIG. 2

**MolePercent**

$Y = 4.25 - 1.12X$
Adjusted R-sqr = 0.7927
Correl. Coef = -0.8928

log(TGLC)

log(TG20:4n6)

FIG. 3

**MolePercent**

TGLC

TG20:4n6

# TG20:4n6

**Reference Cutoff: 0.76**
**Specificity: 1.00**
**Sensitivity: 0.93**

**FIG. 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006031963 A
- US 20060084129 A
- US 20040143461 A
- WO 03005628 A

### Non-patent literature cited in the description

- **PAWLOSKY et al.** *Alcohol,* 2004, vol. 34 (1), 27-33
- **ARAYA et al.** *Clin. Sci.,* 2004, vol. 106 (6), 635-43
- **DE AMEIDA et al.** *Clin. Nutr.,* 2002, vol. 21 (3), 219-23
- **PURI et al.** *Hepatology,* 2007, vol. 46 (4), 1081-90
- **HAUKELAND JW et al.** Systemic inflammation in nonalcoholic fatty liver disease is characterized by elevated levels of CCL2. *J Hepatol.,* June 2006, vol. 44 (6), 1167-74
- **ABIRU S et al.** Serum cytokine and soluble cytokine receptor levels in patients with non-alcoholic steatohepatitis. *Liver Int.,* February 2006, vol. 26 (1), 3945
- **SAVAGE DB et al.** Reversal of diet-induced hepatic steatosis and hepatic insulin resistance by antisense oligonucleotide inhibitors of acetyl-CoA carboxylases 1 and 2. *J Clin Invest.,* March 2006, vol. 116 (3), 817-24
- **HAMMOND LE et al.** Mitochondrial glycerol-3-phosphate acyltransferase-1 is essential in liver for the metabolism of excess acyl-CoAs. *J Biol Chem.,* 08 July 2005, vol. 280 (27), 25629-36
- **HIGASHI Y et al.** Effect of gamma-butyrobetaine on fatty liver in juvenile visceral steatosis mice. *J Pharm Pharmacol.,* April 2001, vol. 53 (4), 527-33
- **MS: CYR D et al.** A GC/MS validated method for the nanomolar range determination of succinylacetone in amniotic fluid and plasma: an analytical tool for tyrosinemia type I. *J Chromatogr B Analyt Technol Biomed Life Sci.,* 17 February 2006, vol. 832 (1), 24-9
- **VOGESER M.** Abstract Liquid chromatography-tandem mass spectrometry-application in the clinical laboratory. *Clin Chem Lab Med.,* February 2003, vol. 41 (2), 117-26
- **KHALIL P et al.** Validation and application of a high-performance liquid chromatographic-based assay for determination of the inosine 5'-monophosphate dehydrogenase activity in erythrocytes. *J Chromatogr B Analyt Technol Biomed Life Sci.,* 23 May 2006
- **FOUASSIER M et al.** Determination of serotonin release from platelets by HPLC and ELISA in the diagnosis of heparin-induced thrombocytopenia: comparison with reference method by [C]-serotonin release assay. *J Thromb Haemost.,* May 2006, vol. 4 (5), 1136-9
- **BADIOU S et al.** Determination of plasma amino acids by fluorescent derivatization and reversed-phase liquid chromatographic separation. *Clin Lab.,* 2004, vol. 50 (3-4), 153-8
- **BRUNELLI T et al.** Comparison of three methods for total homocysteine plasma determination. *Clin Lab.,* 2001, vol. 47 (7-8), 393-7
- **ZINELLU A et al.** Assay for the simultaneous determination of guanidinoacetic acid, creatinine and creatine in plasma and urine by capillary electrophoresis UV-detection. *J Sep Sci.,* March 2006, vol. 29 (5), 704-8
- **JABEEN R et al.** Capillary electrophoresis and the clinical laboratory. *Electrophoresis,* 23 May 2006
- **GAO P et al.** Rapid detection of Staphylococcus aureus by a combination of monoclonal antibody-coated latex and capillary electrophoresis. *Electrophoresis,* May 2006, vol. 27 (9), 1784-9
- **JOHANNESSEN EA et al.** A suspended membrane nanocalorimeter for ultralow volume bioanalysis. *IEEE Trans Nanobioscience,* March 2002, vol. 1 (1), 29-36
- **HERRMANN M et al.** Enzymatically-generated fluorescent detection in microchannels with internal magnetic mixing for the development of parallel microfluidic ELISA. *Lab Chip.,* April 2006, vol. 6 (4), 555-60
- **YANG S et al.** Blood plasma separation in microfluidic channels using flow rate control. *ASAIO J.,* September 2005, vol. 51 (5), 585-90

- **DUPUY AM et al.** Protein biochip systems for the clinical laboratory. *Clin Chem Lab Med.,* 2005, vol. 43 (12), 1291-302
- **PATERSON S et al.** Validation of techniques to detect illicit heroin use in patients prescribed pharmaceutical heroin for the management of opioid dependence. *Addiction,* December 2005, vol. 100 (12), 1832-9
- **BOTTCHER M et al.** Evaluation of buprenorphine CEDIA assay versus GC-MS and ELISA using urine samples from patients in substitution treatment. *J Anal Toxicol.,* November 2005, vol. 29 (8), 769-76
- **JULAK J.** Chromatographic analysis in bacteriologic diagnostics of blood cultures, exudates, and bronchoalveolar lavages. *Prague Med Rep.,* 2005, vol. 106 (2), 175-94
- **BOETTCHER M et al.** Precision and comparability of Abuscreen OnLine assays for drugs of abuse screening in urine on Hitachi 917 with other immunochemical tests and with GC/MS. *Clin Lab.,* 2000, vol. 46 (1-2), 49-52
- **WESTERMANN J et al.** Simple, rapid and sensitive determination of epinephrine and norepinephrine in urine and plasma by non-competitive enzyme immunoassay, compared with HPLC method. *Clin Lab.,* 2002, vol. 48 (1-2), 61-71
- **AOYAGI K et al.** Performance of a conventional enzyme immunoassay for hepatitis C virus core antigen in the early phases of hepatitis C infection. *Clin Lab.,* 2001, vol. 47 (3-4), 119-27
- **HUBL W et al.** A multi-center quality control study of different CA15-3 immunoassays. *Clin Lab.,* 2005, vol. 51 (11-12), 641-5
- **HALLER CA et al.** Comparison of an automated and point-of-care immunoassay to GC-MS for urine oxycodone testing in the clinical laboratory. *J Anal Toxicol.,* March 2006, vol. 30 (2), 106-11
- **BAYER M et al.** Evaluation of a new enzyme-linked immunosorbent assay for the determination of neopterin. *Clin Lab.,* 2005, vol. 51 (9-10), 495-504
- **GROCHE D et al.** Standardization of two immunological HbA1c routine assays according to the new IFCC reference method. *Clin Lab.,* 2003, vol. 49 (11-12), 657-61
- **IVAN D et al.** German KIMS Board. Applicability of recently established reference values for serum insulin-like growth factor 1: A comparison of two assays--an (automated) chemiluminescence immunoassay and an enzyme-linked immunosorbent assay. *Clin Lab.,* 2005, vol. 51 (7-8), 381-7
- **KRAMER KA et al.** Automated spectrophotometric analysis of mitochondrial respiratory chain complex enzyme activities in cultured skin fibroblasts. *Clin Chem.,* November 2005, vol. 51 (11), 2110-6
- **GROCHE D et al.** Standardization of two immunological HbA1c routine assays according to the new IFCC reference method. *Clin Lab.,* 2003, vol. 49 (11-l2), 657-61
- **WOLF PL.** History of diagnostic enzymology: A review of significant investigations. *Clin Chim Acta.,* 24 March 2006
- **MUTCH DM et al.** An integrative metabolism approach identifies stearoyl-CoA desaturase as a target for an arachidonate-enriched diet. *FASEB J.,* April 2005, vol. 19 (6), 599-601
- **STONE SJ et al.** Lipopenia and skin barrier abnormalities in DGAT2-deficient mice. *J Biol Chem.,* 19 March 2004, vol. 279 (12), 11767-76
- **WATKINS SM et al.** Phosphatidylethanolamine-N-methyltransferase activity and dietary choline regulate liver-plasma lipid flux and essential fatty acid metabolism in mice. *J Nutr.,* November 2003, vol. 133 (11), 3386-91
- **WATKINS SM et al.** Lipid metabolome-wide effects of the PPARgamma agonist rosiglitazone. *Lipid Res.,* November 2002, vol. 43 (11), 1809-17
- **STANTON, B. et al.** Interaction of estrogen and 2,3,7,8-tetracholorodibenzo-p-dioxin (TCDD) with hepatic fatty acid synthesis and metabolism of male chickens (Gallus domesticus). *Comp. Biochem. and Physiology,* 2001, vol. 129, 137-150
- **WATKINS, S.M. et al.** Unique Phospholipid Metabolism in Mouse Heart in Response to Dietary Docosahexaenoic or α-Linoleic Acids. *Lipids,* 2001, vol. 36 (3), 247-254
- **BERNHARDT, T.G. et al.** Purification of fatty acid ethyl esters by solid-phase extraction and high-performance liquid chromatography. *J. of Chromatography B,* 1996, vol. 675, 189-196
- **FOLCH, J. ; M. LEES et al.** A simple method for the isolation and purification of total lipides from animal tissues. *J Biol Chem,* 1957, vol. 226 (1), 497-509
- **FOLCH, J. et al.** A simple method for the isolation and purification of total lipids from animal tissues. *J. Biol. Chem.,* 1957, vol. 226, 497-509
- **WATKINS, S. M. et al.** Unique phospholipid metabolism in mouse heart in response to dietary docosahexaenoic or {alpha}-linolenic acids. *Lipids,* 2001, vol. 36, 247-254
- Atlas. Thermo Electron Corporation, 2003